# FASCICULE DE BREVET EUROPEEN

(11) **EP 1 057 826 B1**
(45) Date de publication et mention de la délivrance du brevet: **16.04.2003**
(21) Numéro de dépôt: 00610050.7
(22) Date de dépôt: 22.05.2000
(51) Int. Cl.: C07D 333/58, A61K 31/40, A61K 31/38, A61K 31/44, A61K 31/34, A61P 25/24, A61P 25/28, A61P 9/00, A61P 3/00, C07D 405/06, C07D 307/80, C07D 307/81, C07D 471/04, C07D 405/14, C07D 409/12, C07D 333/66, C07D 407/12, C07D 311/58, C07D 319/20, C07D 405/12

(54) **Dérivés carboxamides dimériques substitués, leur procédé de préparation et les compositions pharmaceutiques qui les contiennent**
Substituierte dimere Carboxamid-Derivate, Verfahren zu ihrer Herstellung und sie enthaltende pharmazeutische Zubereitungen
Substituted dimeric carboxamide derivatives, method for preparing them, and pharmaceutical compositions containing them

(30) Priorité: 19.05.1999 FR 9906331
(43) Date de publication de la demande: 06.12.2000
(73) Titulaire: LES LABORATOIRES SERVIER, 92200 Neuilly sur Seine (FR)
(72) Inventeur: Depreux, Patrick, 59280 Armentieres (FR); Yous, Said, 59000 Lille (FR); Cheve, Gwenael, 59110 La Madeleine (FR); Guillaumet, Gérald, 45650 Saint Jean le Blanc (FR); Viaud, Marie-Claude, 37000 Tours (FR); Larraya, Carlos, 33440 St Vincent de Paul (FR); Bennejean, Caroline, 94220 Charenton le Pont (FR); Delagrange, Philippe, 92130 Issy les Moulineaux (FR); Regard, Pierre, 78150 Le Chesnay (FR); Descamps-Francois, Carole, 59260 Hellemmes (FR)

(56) Documents cités:
- EP-A- 0 447 285
- EP-A- 0 562 956
- EP-A- 0 591 057
- WO-A-96/00720

## Description

La présente invention concerne de nouveaux dérivés dimériques substitués, leur procédé de préparation et les compositions pharmaceutiques qui les contiennent.

Des structures monomériques ayant une affinité pour les récepteurs mélatoninergiques sont décrites dans la littérature (EP 0562956, EP 0447285, EP 0591057).

On connaît dans l'art antérieur des structures dimériques en série naphtalénique (J. Chem. Soc., Dalton Trans., 1979, (10), pp. 1497-502) étudiées pour leurs propriétés de coordination au sein de complexes métalliques, ou en série indolique pour leur activité « curare-like » (Khim.-Farm. Zh., 1984, 18 (1), pp. 29-31). Par ailleurs, les demandes WO 9600720 et WO 9414771 décrivent des structures dimériques mixtes utiles en tant que ligands 5-HT₁ ou en tant qu'intermédiaires de synthèse respectivement.

Les composés de la présente invention, de par leur structure originale, sont nouveaux et présentent des propriétés pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Sécrétions, 1990, 8 (3-4), pp. 264-272) et analgésiques (Pharmacopsychiat., 1987, 20, pp. 222-223) ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sous-types réceptoriels mélatoninergiques.

La présente invention concerne plus particulièrement les composés de formule (I) :

A-G₁-Cy-G₂-Cy'-G₃-B (I)

dans laquelle :
◆ A représente un groupement de formule 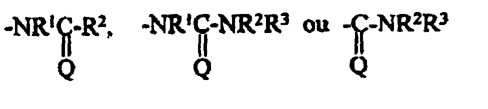 dans lesquelles :
   - Q représente un atome de soufre ou d'oxygène,
   - R¹, R², R³, identiques ou différents, représentent un atome d'hydrogène ou un groupement Rₐ (où Rₐ représente un groupement alkyle (C₁-C₆) linéaire ou ramifié non substitué ou substitué, alkényle (C₂-C₆) linéaire ou ramifié non substitué ou substitué, alkynyle (C₂-C₆) linéaire ou ramifié non substitué ou substitué, cycloalkyle (C₃-C₈) non substitué ou substitué, cycloalkylalkyle (C₃-C₈) linéaire ou ramifié non substitué ou substitué, polyhalogénoalkyle (C₁-C₆) linéaire ou ramifié, aryle, arylalkyle (C₁-C₆) linéaire ou ramifié, arylalkényle (C₂-C₆) linéaire ou ramifié, hétéroaryle, hétéroarylalkyle (C₁-C₆) linéaire ou ramifié ou hétéroarylalkényle (C₂-C₆) linéaire ou ramifié), ou les groupements R² et R³ peuvent également former, avec l'atome d'azote qui les porte un groupement choisi parmi piperazinyle, piperidinyle, ou pyrrolidinyle,

◆ B représente un groupement de formule 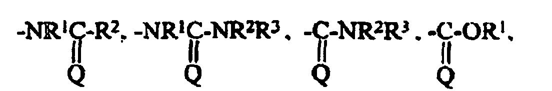 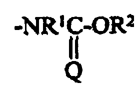 ou -NR²R³ dans lesquelles Q, R¹, R² et R³ sont tels que définis précédemment,
◆ G₁ et G₃, identiques ou différents, représentent une chaîne alkylène contenant de 1 à 4 carbones linéaire ou ramifiée éventuellement substituée par un ou plusieurs groupements, identiques ou différents, choisis parmi hydroxy, carboxy, formyle, Rₐ, ORₐ, COORₐ ou CORₐ (où Rₐ est tel que défini précédemment),
◆ Cy et Cy', différents, représentent
   - une structure cyclique de formule (II) : 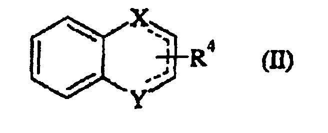
   dans laquelle
   ◆ X et Y, identiques ou différents, représentent un atome de soufre, d'oxygène ou de carbone, ou un groupement CH ou CH₂,
   ◆ R⁴ représente un atome d'hydrogène ou d'halogène ou un groupement CF₃, hydroxy, carboxy, formyle, amino, NHRₐ, NRₐR¹ₐ, NHCORₐ. CONHRₐ, Rₐ, ORₐ, CORₐ ou COORₐ, (où Rₐ est tel que défini précédemment et R¹ₐ peut prendre toutes les valeurs de Rₐ),
   ◆ la représentation ---- signifie que les liaisons sont simples ou doubles, étant entendu que la valence des atomes est respectée,
   où G₂ substitue le cycle benzénique, et G₁ substitue le cycle contenant X et Y dans le cas de Cy
   et G₂ substitue le cycle benzénique et G₃ substitue le cycle contenant X et Y dans le cas de Cy',

- ou une structure cyclique de formule (III) : 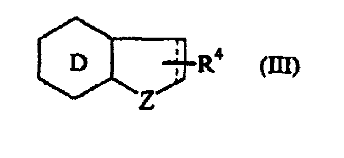
dans laquelle :
* Z représente un atome de soufre ou d'oxygène, ou un groupement CH₂, NH, NSO₂Ph ou NRₐ (où Rₐ est tel que défini précédemment),
* D représente un cycle benzénique ou pyridinique,
* R⁴ est tel que défini précédemment,
* la représentation ---- signifie que la liaison est simple ou double, étant entendu que la valence des atomes est respectée,
où G₂ substitue le cycle D, et G₁ substitue le cycle contenant Z dans le cas de Cy
et G₂ substitue le cycle D et G₃ substitue le cycle contenant Z dans le cas de Cy'.
les deux cycles Cy et Cy' des composés de formule (I), différents, étant représentés tous les deux par une structure de formule (II), tous les deux par une structure de formule (III), ou l'un par une structure de formule (II) et l'autre par une structure de formule (III),
◆ G₂ représente une chaîne de formule (IV) : 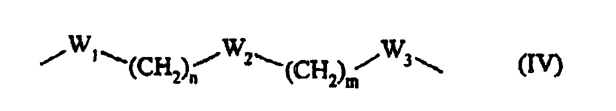 dans laquelle :
   - W₁, W₂ et W₃, identiques ou différents, représentent une liaison, un atome d'oxygène ou de soufre, ou un groupement CH₂, CHRₐ, NH ou NRₐ (où Rₐ est tel que défini précédemment).
   - n représente un entier tel que 0 ≤ n ≤ 6,
   - m représente un entier tel que 0 ≤ m ≤ 6,
   étant entendu que l'on ne peut pas avoir deux hétéroatomes consécutifs et que la chaîne de formule (IV) ainsi définie peut comporter une ou plusieurs insaturations,
   étant entendu que :
   - par « aryle », on entend les groupements naphtyle, phényle et biphényle,
   - par « hétéroaryle », on entend tout groupement mono ou bicyclique, saturé ou insaturé contenant 5 à 10 chaînons et contenant 1 à 3 hétéroatomes choisis parmi azote, soufre ou oxygène, les groupements « aryles » et « hétéroaryles » pouvant être substitués par un ou plusieurs radicaux, identiques ou différents, choisis parmi hydroxy, carboxy, alkoxy (C₁-C₆) linéaire ou ramifié, alkyle (C₁-C₆) linéaire ou ramifié, polyhalogénoalkyle(C₁-C₆) linéaire ou ramifié, formyle, cyano, nitro, amino, alkylamino (C₁-C₆) linéaire ou ramifié, dialkylamino (C₁-C₆) linéaire ou ramifié ou atomes d'halogène,
   - le terme «substitué» affecté aux expressions « alkyle », « alkényle », et « alkynyle » signifie que ces groupements sont substitués par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié ou atomes d'halogène,
   - le terme « substitué » affecté aux expressions « cycloalkyle » et « cycloalkylalkyle » signifie que la partie cyclique de ces groupements est substituée par un ou plusieurs radicaux, identiques ou différent, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié ou atomes d'halogène,
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels :
- Cy et Cy', différents, représentent une structure cyclique de formule (II) comme le naphtalène, le tétrahydronaphtalène, les noyaux 1,4-benzodioxine ou chromane par exemple,
- Cy et Cy', différents, représentent une structure cyclique de formule (III) comme l'indole, l'azaindole, le benzothiophène ou le benzofurane par exemple,
- Cy représente une structure cyclique de formule (II) et Cy' représente une structure cyclique de formule (III).

De façon avantageuse, l'invention concerne les composés de formule (I) pour lesquels G₂ représente une liaison simple,
ou un groupement -W₄-(CH₂)ₚ-W'₄- (où W₄ et W'₄, identiques ou différents, représentent un atome d'oxygène ou de soufre, ou un groupement NH ou NRₐ, et p représente un entier tel que 1 ≤ p ≤ 12) comme par exemple le groupement -O-(CH₂)ₚ-O- (où p est tel que défini précédemment),
ou un groupement de formule -W₄-(CH₂)_{p'}-W'₄-(CH₂)_{p''}-W"₄- (où W₄, W'₄, et W"₄, identiques ou différents, représentent un atome d'oxygène ou de soufre, ou un groupement NH ou NRₐ, et p' et p" sont deux entiers tels que 2 ≤ p'+p" ≤ 12) comme par exemple le groupement -O-(CH₂)ₚ-O-(CH₂)_{p"}-O- (où p' et p" sont tels que définis précédemment).

Les substituants A et B préférés de l'invention sont les groupements NR¹C(Q)R², NR¹C(Q)NR²R³ ou C(Q)NR²R³ et plus particulièrement les groupements NR¹COR² ou CONR²R³.

Encore plus particulièrement, l'invention concerne les composés de formule (I) qui sont :
- le *N*-(2-{7-[2-({3-[2-(acétylamino)éthyl]-1-benzofuran-5-yl}oxy)éthoxy]-1-naphtyl} éthyl)acétamide,
- le *N*-(2-{5-[2-({8-[2-(acétylamino)éthyl]-2-naphtyl}oxy)éthoxy]-1-benzofuran-3-yl}éthyl)-2-furamide,
- le *N*-(2-{5-[2-({8-[2-(acétylamino)éthyl]-2-naphtyl}oxy)éthoxy]-*1H*-pyrrolo[*2,3-b*] pyridin-3-yl}éthyl)cyclopropanecarboxamide,
- le *N*-(2-{7-[3-({3-[2-(acétylamino)éthyl]-1-benzothiophèn-5-yl}oxy)propoxy]-1-naphtyl}éthyl)acétamide.
- le *N*-[2-(5-{[6-({8-[2-(acétylamino)éthyl]-2-naphtyl}oxy)hexyl]oxy}-*1H*-pyrrolo [*2,3-b*]pyridin-3-yl)éthyl]acétamide.
- le *N*-(2-{7-[4-({3-[2-(acétylamino)éthyl]-1-benzofuran-5-yl}oxy)butoxy]-1-naphtyl} éthyl)acétamide,
- le *N*-{2-[5-[4-({8-[2-(acétylamino)éthyl]-2-naphtyl}oxy)butoxy]-1-(phénylsulfonyl)-1*H*-indol-3-yl]éthyl}acétamide,
- le *N*-(2-{7-[4-({8-[2-(acétylamino)éthyl]-2-naphthyl}oxy)butoxy]-1,2,3,4-tétrahydro-1-naphtalényl}éthyl)acétamide,
- le *N*-(2-{5-[4-({3-[2-(acétylamino)éthyl]-3a,7a-dihydro-1-benzofuran-5-yl}oxy) butoxy]-1*H*-indol-3-yl}éthyl)acétamide,
- le *N*-(2-{7-[4-({3-[2-(acétylamino)éthyl]-1-benzothien-5-yl}oxy)butoxy]-1-naphtyl} éthyl)acétamide,
- le *N*-(2-{5-[4-({3-[2-(acétylamino)éthyl]-1-benzothien-5-yl}oxy)butoxy]-1*H*-indol-3-yl}éthyl)acétamide,
- le *N*-(2-{5-[4-({3-[2-(acétylamino)éthyl]-1-benzothien-5-yl}oxy)butoxy]-1*H*-pyrrolo [2,3-*b*]pyridin-3-yl}éthyl)acétamide,
- le *N*-(2-{5-[4-({3-[2-(acétylamino)éthyl]-1-benzofuran-5-yl}oxy)butoxy]-1*H*-pyrrolo [2,3-*b*]pyridin-3-yl}éthyl)acétamide,
- le *N*-(2-{5-[4-({3-[2-(acétylamino)éthyl]-1*H*-indol-5-yl}oxy)butoxy]-1*H*-pyrrolo[2,3-*b*]pyridin-3-yl}éthyl)acétamide,
- le *N*-[2-(7-{3-[2-(acétylamino}éthyl]-1-benzofuran-5-yl}-1-naphtyl)éthyl]acétamide,
- le *N*-[3-(5-{8-[2-(acétylamino)éthyl]-2-naphtyl}-*1H*-pyrrolo[*2,3-b*]pyridin-3-yl) propyl]heptanamide,
- le *N*-[2-(7-{3-[2-(acétylamino)éthyl]-1-benzothien-5-yl}-1-naphtyl)éthyl]acétamide,
- le *N*-[2-(5-{8-[2-(acétylamino)éthyl]-2-naphtyl}-1*H*-pyrrolo[2,3-*b*]pyridin-3-yl) éthyl]acétamide,
- le *N*-[2-(5-{3-[2-(acétylamino)éthyl]-1-benzofuran-5-yl}-1-benzothien-3-yl)éthyl] acétamide,
- le *N*-[2-(5-{3-[2-(acétylamino)éthyl]-1-benzofuran-5-yl}-1*H*-indol-3-yl)éthyl] acétamide,
- le *N*-[2-(5-{3-[2-(acétylamino)éthyl]-1-benzofuran-5-yl}-1*H*-pyrrolo[2,3-*b*]pyridin-3-yl)éthyl]acétamide,
- le *N*-[2-(5-{3-[2-(acétylamino)éthyl]-1*H*-indol-5-yl}-1*H*-pyrrolo[2,3-*b*]pyridin-3-yl)éthyl]acétamide,
- le *N*-[2-(5-{3-[2-(acétylamino)éthyl]-1-benzothien-5-yl}-1*H*-pyrrolo[2,3-*b*]pyridin-3-yl)éthyl]acétamide.

Les énantiomères, diastéréoisomères ainsi que les sels d'addition à un acide ou une base pharmaceutiquement acceptable des composés préférés de l'invention font partie intégrante de l'invention.

La présente invention concerne également le procédé de préparation des composés de formule (I) caractérisé en ce que l'on utilise comme produit de départ le composé de formule (V):

A-G₁-Cy-OMe (V)

dans laquelle A, G₁ et Cy sont tels que définis dans la formule (I),
que l'on soumet à une déméthylation en utilisant des agents classiques comme HBr, AlCl₃, AlBr₃, BBr₃ ou des systèmes binaires acide de Lewis / nucléophile comme AlCl₃ / PhCH₂SH ou BBr₃ / Me₂S par exemple, pour obtenir le composé de formule (VI) :

A-G₁-Cy-OH (VI)

dans laquelle A, G₁ et Cy sont définis comme précédemment,
◆ qui est transformé, de façon classique,
   - par action du *N,N*-diméthylthiocarbamate de sodium par exemple, en thiol correspondant de formule (VII) :

      A-G₁-Cy-SH (VII)

      dans laquelle A, G₁ et Cy sont tels que définis précédemment,
   - ou en dérivé aminé correspondant de formule (VIII) :

      A-G₁-Cy-NHR'ₐ (VIII)

      dans laqucllc A, G₁ et Cy sont définis comme précédemment et R'ₐ peut prendre toutes les valeurs de Rₐ tel que défini dans la formule (I) et peut également représenter un atome d'hydrogène,
   les composés de formule (VI), (VII) et (VIII) représentant le composé de formule (IX) :

   A-G₁-Cy-W₄H (IX)

   dans laquelle W₄ représente un atome d'oxygène ou de soufre, ou un groupement NH ou NRₐ (où Rₐ est défini comme précédemment),
   composé de formule (IX) sur lequel on condense :
◆ un composé de formule (X) : 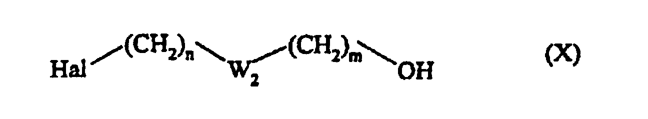 dans laquelle Hal représente un atome de brome, de chlore ou d'iode, et n, W₂ et m sont tels que définis dans la formule (I), (étant entendu que l'on ne peut pas avoir deux hétéroatomes consécutifs et que la chaîne ainsi définie peut comporter une ou plusieurs insaturations),
◆ ou un composé de formule (XI) : 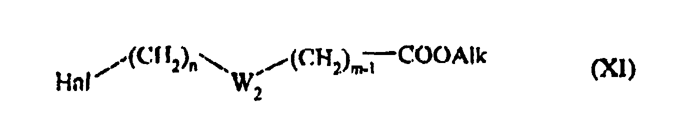 dans laquelle Hal, n, m et W₂ sont définis comme précédemment et Alk représente un radical alkyle (étant entendu que l'un ne peut pas avoir deux hétéroatomes consécutifs et que la chaîne ainsi définie peut comporter une ou plusieurs insaturations), suivi d'une réduction.
   pour conduire au composé de formule (XII) :

   A-G₁-Cy-W₄-(CH₂)ₙ-W₂-(CH₂)ₘ-OH (XII)

   dans laquelle A, G₁, Cy, W₄, n, m et W₂ sont définis comme précédemment (étant entendu que l'on ne peut pas avoir deux hétérontomes consécutifs dans l'enchaînement -W₄-(CH₂)ₙ-W₂-(CH₂)ₘ-OH et que la chaîne ainsi définie peut comporter une ou plusieurs insaturations).
   dont la fonction hydroxyle est transformée classiquement en groupe partant comme par exemple un mésylate, un tosylate, nu un dérivé halogéné, pour conduire au composé de formule (XII') :

   A-G₁-Cy-W₄-(CH₂)ₙ-W₂-(CH₂)ₘ-E (XIIʹ)

   dans laquelle A, G₁, Cy, W₄, n, W₂ et m sont définis comme précédemment et E représente un groupement mésyle ou tosyle ou un atome d'halogéne,
   sur lequel on fait agir un composé de formule (XIII) :

   B-G₃-Cy'-W'₄H (XIII)

   dans laquelle B, G₃ et Cy' sont définis comme dans la formule (I) et W'₄ peut prendre les mêmes valeurs que W₄ défini précédemment,
   pour conduire au composé de formule (I/a), cas particulier des composés de formule (I) :

   A-G₁-Cy-W₄-(CH₂)ₙ-W₂-(CH₂)ₘ-W'₄-Cy'-G₃-B (I/a)

   dans laquelle A, G₁, Cy, Cy', W₄, n, W₂, m, W'₄, G₃ et B sont tels que définis précédemment.
◆ ou transformé en utilisant par exemple le phényl bis (trifluorométhane-sulfonimide) en milieu basique en trifluorométhane sulfonate correspondant de formule (XIV):

   A-G₁-Cy-OSO₂CF₃ (XIV)

   dans laquelle A, G₁ et Cy sont définis comme précédemment.
   - sur lequel on fait agir, dans des conditions de catalyse par un dérivé du palladium approprié, un dérivé de l'acide borique (R_{b}B(OH)₂) ou un dérivé de l'étain (R_{b}SnBu₃) (où R_{b} représente un groupement de formule (XV) :

      B-G₃-Cy'-W₃-(CH₂)ₘ-W₂-(CH₂)ₙ-CH₂- (XV)

      dans laquelle B, G₃, Cy', W₃, m, W₂ et n sont tels que définis précédemment, étant entendu que l'on ne peut pas avoir deux hétéroatomes consécutifs dans l'enchaînement -W₃-(CH₂)ₘ-W₂- et que la chaîne ainsi définie peut comporter une ou plusieurs insaturations).
      pour conduire au composé de formule (I/b), cas particulier des composés de formule (I) :

      A-G₁-Cy-CH₂-(CH₂)ₙ-W₂-(CH₂)ₘ-W₃-Cy'-G₃-B (I/b)

      dans laquelle A, G₁, Cy, Cy', n, W₂, m, W₃, G₃ et B sont définis comme précédemment (étant entendu que l'on en peut pas avoir deux hétéroatomes consécutifs dans l'enchaînement -W₂-(CH₂)ₘ-W₃- et que la chaîne ainsi définie peut comporter une ou plusieurs insaturations),
      les composés de formule (I/c), cas particulier des composés de formule (I) :

      A-G₁-Cy-W₁-(CH₂)ₙ-W₂-(CH₂)ₘ-CH₂-Cy'-G₃-B (I/c)

      dans laquelle A, G₁, Cy, Cy', W₁, n, W₂, m, G₃ et B sont définis comme précédemment (étant entendu que l'on ne peut pas avoir deux hétéroatomes consécutifs dans l'enchaînement -W₁-(CH₂)ₙ-W₂- et que la chaîne ainsi définie peut comporter une ou plusieurs insaturations),
      étant obtenus selon un mode opératoire similaire à partir du composé de formule (XIV') :

      B-G₃-Cy'-OSO₂CF₃ (XIVʹ)

      dans laquelle B, G₃ et Cy' sont tels que définis précédemment.
   - ou que l'on place, dans des conditions de couplage en utilisant des dérivés du Nickel ou du palladium par exemple, en présence d'un composé de formule (XIV') afin de conduire au composé de formule (I/d), cas particulier des composés de formule (I) :

      A-G₁-Cy-Cy'-G₃-B (I/d)

      dans laquelle A, G₁, Cy, Cy', G₃ et B sont tels que définis précédemment,
      l'ensemble des composés (I/a) à (I/d) formant le composé de formule (1) qui peut être purifié si on le désire par une technique classique de purification, dont on sépare éventuellement les isomères selon une technique classique de séparation et que l'on transforme, le cas échéant, en leurs sels d'addition à un acide ou à une base pharmaceutiquement acceptable.
      Les composés de formule (V) sont aisément accessibles à l'homme du métier selon des méthodes décrites dans la littérature.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une haute affinité pour les récepteurs de la mélatonine et possédaient d'importantes activités sur le système nerveux central ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des pathologies du systèmc digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques. de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonniéres et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per, ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon. Les préparations suivantes conduisent à des composés de l'invention ou à des intermédiaires de synthèse utiles dans la préparation de l'invention.

### Préparation 1 : N-[2-(7-Hydroxy-1-naphtyl)éthyl]-acétamide

Sous atmosphère inerte, 27,5 mmol du complexe tribromure de bore/diméthylsulfure sont dissoutes dans 100 ml de dichlorométhane et agitées pendant 15 minutes à température ambiante. Une solution de 13,7 mmol de *N*-[2-(7-méthoxy-1-naphtyl)éthyl]acétamide dans 50 ml de dichlorométhane est ajoutée, et le milieu réactionnel est porté à reflux pendant 30 heures. Après refroidissement, la réaction est hydrolysée avec précaution et le dichlorométhane est évaporé. Le milieu est alors extrait à l'acétate d'éthyle, les phases organiques regroupées sont lavées par une solution aqueuse de bicarbonate de potassium 1M. La phase organique est séchée sur sulfate de magnésium, et concentrée pour conduire au composé du titre. Solide blanc.
Point de fusion : 125-126°C

En procédant comme dans la Préparation 1 à partir du substrat approprié, on obtient les Préparations 2 à 35 :

### Préparation 2 : N-[2-(5-Hydroxy-1-benzofuran-3-yl)éthyl]acétamide

### Préparation 3 : N-[2-(5-Hydroxy-1-benzofuran-3-yl)éthyl]cyclopropane carboxamide

### Préparation 4 : N-[2-(5-Hydroxy-1-benzofuran-3-yl)éthyl]-2-faramide

### Préparation 5 : N-[2-(7-Hydroxy-1-naphtyl)éthyl]benzamide

### Préparation 6 : N-[2-(7-Hydroxy-1-naphtyl)éthyl]-3-buténamide

### Préparation 7 : N-[2-(5-Hydroxy-1-benzofuran-3-yl)éthyl]-2-méthylpropanamide

### Préparation 8 : N-[2-(7-Hydroxy-1-naphtyl)éthyl]-2-phénylacétamide

### Préparation 9 : N-[2-(5-Hydroxy-1-benzothiophèn-3-yl)éthyl]-acétamide

### Préparation 10 : N-[2-(5-Hydroxy-1H-pyrrolo[2,3-b]pyridin-3-yl)éthyl]cyclopropane carboxamide

### Préparation 11 : N-[2-(5-Hydroxy-1H-indol-3-yl)éthyl]acétamide

### Préparation 12 : N-[2-(5-Hydroxy-1H-pyrrolo[2,3-b]pyridin-3-yl)éthyl]acétamide

### Préparation 13 : N-[2-(7-Hydroxy-1-naphtyl)éthyl]cyclobutane carboxamide

### Préparation 14 : 2,2,2-Trifluoro-N-[2-(7-hydroxy-1-naphtyl)éthyl]acétamide

### Préparation 15 : N-[(6-Hydroxy-2H-chromèn-3-yl)méthyl]butanamide

### Préparation 16 : N-[(6-Hydroxy-2H-chromèn-3-yl)méthyl]acétamide

### Préparation 17 : N-[(7-Hydroxy-1,4-benzodioxin-2-yl)méthyl]-N'-propylurée

### Préparation 18 : N-[(7-Hydroxy-1,4-benzodioxin-2-yl)méthyl]acétamide

### Préparation 19 : N-[2-(7-Hydroxy-1-naphtyl)éthyl]furamide

### Préparation 20 : N-[2-(2-Benzyl-5-hydroxy-1H-pyrrolo[2,3-b]pyridin-3-yl)éthyl]acétamide

### Préparation 21 : N-[2-(5-Hydroxy-1-benzothiophèn-3-yl)éthyl]cyclohexane carboxamide

### Préparation 22 : N-Hexyl-2-(5-hydroxy-1-benzofuran-3-yl)acétamide

### Préparation 23 : 2,2,2-Trifluoro-N-[2-(5-hydroxy-1-benzothiophèn-3-yl)éthyl] acétamide

### Préparation 24 : N-[2-(6-Hydroxy-3,4-dihydro-2H-chromèn-4-yl)éthyl]acétamide

### Préparation 25 : N-[2-[7-Hydroxy-1,2,3,4-tétrahydro-1-naphtalènyl)éthyl]acétamide

### Préparation 26: N-[2-(7-Hydroxy-1,2,3,4-tétrahydro-1-naphtalènyl)éthyl] cyclopropane carboxamide

### Préparation 27 : N-[2-(7-Hydroxy-1-naphtyl)éthyl]heptanamide

### Préparation 28 : N-[2-(5-Hydroxy-1H-indol-3-yl)éthyl]cyclobutane carboxamide

### Préparation 29 : 4-(7-Hydroxy-1-naphtyl)-N-isopropylbutanamide

### Préparation 30 : N-[2-(5-Hydroxy-1-benzofuran-3-yl)éthyl]-N'-phénylurée

### Préparation 31 : N-Benzyl-2-(5-hydroxy-1-benzothiophèn-3-yl)acétamide

### Préparation 32 : N-[2-(5-Hydroxy-1H-indèn-3-yl)éthyl]pentanamide

### Préparation 33 : 3-(5-Hydroxy-1-benzofuran-3-yl)-N-méthylpropanamide

### Préparation 34 : N-[2-(5-Hydroxy-1H-pyrrolo[2,3-b]pyridin-3-yl)éthyl]-N'-méthylurée

### Préparation 35 : 4-(5-Hydroxy-1H-indol-3-yl)-N-méthylbutanamide

### Préparation 36 : N-[2-(5-Mercapto-1-henzofuran-3-yl)éthyl]acétamide

A une solution d'hydroxyde de potassium (10 mmol) dissoute dans 15 ml d'eau et 16 ml de tétrahydrofurane, on additionne le produit obtenu au stade A (9 mmol) en maintenant l'agitation. La solution est refroidie à l'aide d'un bain de glace et de sel et on ajoute goutte à goutte le chlorure diméthylthiocarbamoyle (9 mmol) en solution dans le tétrahydrofurane (15 ml) sous agitation. Après une demi-heure d'agitation en maintenant le froid, le milieu réactionnel est extrait au chloroforme. Les phases organiques sont regroupées, séchées sur sulfate de magnésium, filtrées puis concentrées sous pression réduite. Le résidu est repris dans le diphényléther (10 ml) et porté au reflux pendant une heure sous atmosphère d'azote. Le diphényléther est évaporé sous pression réduite jusqu'à obtention d'une solution d'environ 2 ml. Les 2 ml de distillat encore chauds sont versés avec précaution dans 50 ml d'hexane pour donner après refroidissement un solide isolé par filtration. Le solide ainsi collecté est additionné à une solution d'hydroxyde de potassium (380 mg) dissous dans un mélange eau/méthanol (1 ml/10 ml). La solution est portée au reflux pendant 12 heures puis refroidie et concentrée sous pression réduite. Le résidu est repris avec 20 ml de chloroforme et extrait 3 fois à l'eau. La phase organique est séchée sur sulfate de magnésium, filtrée et concentrée sous pression réduite. Le résidu est chromatographié sur gel de silice pour conduire au produit du titre.

### Préparation 37 : N-[2-(5-Mercapto-1-benzothiophèn-3-yl)éthyl]butanamide

### Stade A : N-[2-(5-Hydroxy-1-benzothiophèn-3-yl)éthyl]butanamide

On procède comme dans la Préparation 1 à partir du *N*-[2-(5-Méthoxy-1-benzothiophèn-3-yl)éthyl]butanamide.

### Stade B : N-[2-(5-Mercapto-1-benzothiophèn-3-yl)éthyl]butanamide

On procède comme dans la Préparation 36 à partir du composé obtenu au stade A.

### Préparation 38 : N-(2-{5-Mercapto-2-[4-(trifluorométhyl)benzyl]-1-benzothiophèn-3-yl}éthyl)acétamide

### Stade A : N-(2-{5-Hydroxy-2-[4-(trifluorométhyl)benzyl]-1-benzothiophèn-3-yl}éthyl)acétamide

On procède comme dans la Préparation 1 à partir du *N*-(2-{5-Méthoxy-2-[4-(trifluorométhyl)benzyl]-1-benzothiophèn-3-yl}éthyl)acétamide.

### Stade B : N-(2-{5-Mercapto-2-[4-(trifluorométhyl)benzyl]-1-benzothiophèn-3-yl}éthyl)acétamide

On procède comme dans la Préparation 36 à partir du composé obtenu au stade A.

### Préparation 39 : N-[2-(7-Mercapto-1,2,3,4-tétrahydro-1-naphtalényl)éthyl] cyclopropane carboxamide

On procède comme dans la Préparation 36 à partir du composé obtenu dans la Préparation 26.

### Préparation 40: N-[2-(5-Amino-1-benzofuran-3-yl)éthyl]acétamide

### Stade A: N-[2-(5-Bromo-1-benzofuran-3-yl)éthyl]acétamide

Dans un tricol de 150 ml équipé d'une ampoule à brome, d'un réfrigérant surmonté d'un tube rempli de chlorure de calcium, et d'un agitateur mécanique, on verse de la triphénylphosphine (10 mmol) et de l'acétonitrile (70 ml). La solution est refroidie à l'aide d'un bain de glace en maintenant l'agitation et on additionne le brome (10 mmol). A la fin de l'addition, le bain de glace est retiré puis on ajoute le produit obtenu dans la Préparation 2 (8 mmol). Le mélange réactionnel est agité à 60-70°C jusqu'à disparition du produit de départ. En fin de réaction, le mélange est filtré puis le filtrat est concentré sous pression réduite. Le résidu est repris dans l'acétate d'éthyle, lavé à l'enu puis avec une solution saturée d'hydrogénocarbonate de potassium, et encore une fois à l'eau puis séché sur sulfate de magnésium et concentré sous pression réduite. Le résidu est filtré sur gel de silice pour conduire au produit du titre.

### Stade B : N-[2-(5-lodo-1-benzofuran-3-yl)éthyl]acétamide

Un mélange du produit obtenu au stade A (2 mmol), d'iodure de potassium (30 mmol) et d'iodure de cuivre I (10 mmol) dans l'hexaméthyl phosphoramide (6 ml) est chauffé à 150-160°C avec agitation sous atmosphère d'azote jusqu'à ce qu'un taux de conversion de 90 % soit atteint. On ajoute alors de l'acide chlorhydrique dilué puis de l'éther et la mixture est alors filtrée pour éliminer les sels de cuivre I insolubles. La phase organique est séparée, lavée avec une solution de sulfite de sodium, de l'eau, séchée sur sulfate de magnésium et évaporée pour donner un résidu que l'on chromatographie sur gel de silice pour conduire au produit du titre.

### Stade C : N-[2-(5-Vinyl-1-benzofuran-3-yl)éthyl]acétamide

15 mmol du produit obtenu au stade B, 16 mmol de vinyl tributylétain et 0,43 mmol de (triphénylphosphine) palladium tetrakis, sont portés sous agitation à 110°C pendant 3 heures dans 30 ml de *N*-méthylpyrrolidinone. Après évaporation du solvant, le résidu est repris dans 20 ml de dichlorométhane et traité par une solution aqueuse à 10 % de fluorure de potassium. Après extraction, concentration sous pression réduite et chromatographie sur gel de silice, on obtient le produit du titre pur.

### Stade D : N-[2-(5-Formyl-1-benzofuran-3-yl)éthyl]acétamide

A une solution de 10 mmol du produit obtenu dans le stade C dans un mélange de 50 ml de dioxane et 25 ml d'eau sont ajoutés à température ambiante 1,10 g de tétroxyde d'osmium dans le 2-méthyl-2-propanol, puis 8,70 g de Periodate de sodium. Après agitation une nuit à température ambiante, la suspension est filtrée, le filtrat concentré sous pression réduite. Le résidu obtenu est repris dans le dichlorométhane. La phase organique est lavée avec de l'eau, séchée et évaporée. Le résidu est purifié par chromatographie sur gel de silice pour conduire au produit du titre.

### Stade E : Acide 3-[2-(acétylamino)éthyl]-1-benzofuran-5-carboxylique

A une solution de 6.88 mmol du produit obtenu dans le stade D dans 30 ml d'acétone sont ajoutés à température ambiante 2,7 g de permanganate de potassium dans 50 ml d'un mélange acétone/eau (50/50). La solution est agitée 2 heures à température ambiante puis filtrée. Le filtrat est concentré sous pression réduite et chromatographié sur gel de silice pour conduire au produit du titre.

### Stade F : Chlorure de l'acide 3-[2-(acétylamino)éthyl]-1-benzofuran-5-carboxylique

5 mmol du produit obtenu dans le stade E sont dissoutes dans 40 ml de chlorure de thionyle. Après agitation sous atmosphère inerte pendant 1 heure, le chlorure de thionyle est évaporé sous pression réduite pour conduire au produit du titre.

### Stade G ; N-[2-(5-Amino-1-benzofuran-3-yl)éthyl]acétamide

Une solution du produit obtenu dans le stade F (20 mmol) dans le dichlorométhane (30 ml) contenant du bromure de tétrabutyl ammonium (20 mg) est refroidie dans un bain de glace. Après addition de l'azoture de sodium (25 mmol) dissous dans 5 ml d'eau la solution est agitée vigoureusement à 0°C pendant 2 heures. La phase organique est séparée, lavée à l'eau (2 x 5 ml) et séchée sur sulfate de magnésium. Après filtration, on ajoute l'acide trifluoroacétique (30 mmol) et la solution est agitée sous reflux pendant 60 heures. Après refroidissement, la phase organique est lavée avec une solution saturée d'hydrogénocarbonate de sodium (2 x 5 ml) et concentrée sous pression réduite. Le résidu est alors repris dans le méthanol (20 ml) et on ajoute de l'eau (80 ml) puis du carbonate de potassium (30 mmol). Après agitation à température ambiante pendant 20 heures, le milieu réactionnel est concentré sous pression réduite jusqu'à un volume de 60 ml environ puis extrait 3 fois à l'éther (3 x 50 ml). Après séchage sur sulfate de sodium, la phase organique est filtrée puis évaporée sous pression réduite. Le résidu est chromatographié sur gel de silice pour conduire nu produit du titre.

### Préparation 41 : N-[2-(5-Amino-1-benzothiophèn-3-yl)éthyl]pentanamide

### Stade A : N-[2-(5-Hydroxy-1-benzothiophèn-3-yl)éthyl]pentanamide

On procède comme dans la Préparation 1 à partir du *N*-[2-(5-Méthoxy-1-benzothiophèn-3-yl)éthyl]pentanamide.

### Stade B : N-[2-(5-Amino-1-benzothiophèn-3-yl)éthyl]pentanamide

On procède comme dans la Préparation 40 à partir du composé obtenu au stade A.

### Préparation 42 : N-{2-[5-Amino-2-(3-méthoxybenzyl)-1-benzofuran-3-yl]éthyl} acétamide

### Stade A : N-{2-[5-Hydroxy-2-(3-méthoxyhenzyl)-1-benzofuran-3-yl]éthyl} acétamide

On procède comme dans la Préparation 1 à partir du *N*-{2-[5-méthoxy-2-(3-méthoxybenzyl)-1-benzofuran-3-yl]éthyl}acétamide.

### Stade B : N-{2-[5-Amino-2-(3-méthoxybenzyl)-1-benzofuran-3-yl]éthyl} acétamide

On procède comme dans la Préparation 40 à partir du composé obtenu au stade A.

### Préparation 43 : N-[2-(5-Amino-1-benzofuran-3-yl)éthyl]-2-furamide

On procède comme dans la Préparation 40 à partir du composé obtenu dans la Préparation 4.

### Préparation 44 : N-[2-(5-Amino-1-benzofuran-3-yl)éthyl]-N'-cyclopropylurée

### Stade A : N-[2-(5-Hydroxy-1-benzofuran-3-yl)éthyl]-N'-cyclopropylurée

On procède comme dans la Préparation 1 à partir du *N*-[2-(5-méthoxy-1-benzofuran-3-yl)éthyl]-N'-cyclopropylurée

### Stade B : N-[2-(5-Amino-1-benzofuran-3-yl)éthyl]-N'-cyclopropylurée

On procède comme dans la Préparation 40 à partir du composé obtenu au stade A.

### Préparation 45 : 3-[2-(Acétylamino)éthyl]-1-benzofuran-5-yl trifluorométhanesulfonate

A une solution de 0,07 mol du composé obtenu dans la Préparation 2 dans un litre de dichlorométhane sont ajoutés 60 ml de triéthylamine. Le milieu réactionnel est porté à reflux jusqu'à solubilisation, puis 0,1 mol de phényl bis (trifluorométhanesulfonimide) et 0,75 mol de carbonate de potassium sont ajoutés. Après 4 heures à reflux, le milieu est lavé par un litre d'hydrogénocarbonate de sodium 1M puis par un litre d'acide chlorhydrique 1M. La phase organique est séchée, concentrée et purifiée par chromatographie sur gel de silice pour conduire au produit du titre.

Les Préparations 46 à 70 sont obtenues en procédant comme dans la Préparation 45.

### Préparation 46 : 8-[2-(Acétylamino)éthyl]-2-naphtyl trifluorométhanesulfonate

Produit de départ : Préparation 1

### Préparation 47 : 3-{2-[(Cyclopropylcarbonyl)amino}éthyl)-1-benzothiophèn-5-yl trifluorométhanesulfonate

Produit de départ : *N*-[2-(5-Hydroxy-1-benzothiophèn-3-yl)éthyl]cyclopropanecarboxamide, obtenu en procédant comme dans la Préparation 1 à partir du *N*-[2-(5-méthoxy-1-benzothiophèn-3-yl)éthyl]cyclopropane carboxamide.

### Préparation 48 : N-(2-{[(Méthylamino)carbonyl]amino}éthyl)-2-naphtyl trifluorométhanesulfonate Produit de départ :N-[2-(7-Hydroxy-1-naphtyl)éthyl]-N'-méthylurée obtenu en procédant comme dans la Préparation 1 à partir de la N-[2-(7-méthoxy-1-naphtyl)éthyl]-N'-méthylurée.

### Préparation 49 : 3-{2-[(Anilinocarbonyl)amino]éthyl}-1-benzofuran-5-yl trifluorométhanesulfonate

Produit de départ : Préparation 30

### Préparation 50 : 3-[2-(2-Furoylamino)éthyl]-1-benzothiophèn-5-yl trifluorométhanesulfonate

Produit de départ : *N*-[2-(5-Hydroxy-1-benzothiophèn-3-yl)éthyl]-2-furamide, obtenu en procédant comme dans la Préparation 1 à partir du *N*-[2-(5-méthoxy-1 -benzothiophèn-3-yl)éthyl]-2-furamide.

### Préparation 51 : 3-[2-(Benzylamino)-2-oxoéthyl]-1H-indol-5-yl trifluorométhanesulfonate

Produit de départ : *N*-Benzyl-2-(5-hydroxy-*1H*-indol-3-yl)acétamide, obtenu en procédant comme dans la Préparation 1 à partir du *N*-benzyl-2-(5-méthoxy-*1H*-indol-3-yl)acétamide.

### Préparation 52 : 3-[3-(Benzoylamino)propyl]-1H-indol-5-yl trifluorométhanesulfonate

Produit de départ : *N*-[3-(5-Hydroxy-*1H*-indol-3-yl)propyl]benzamide, obtenu en procédant comme dans la Préparation 1 à partir du *N*-[3-(5-méthoxy-*1H*-indol-3-yl)propyl]benzamide.

### Préparation 53 : 3-[2-(Isobutyrylamino)éthyl)-1-benzothiophèn-5-yl trifluorométhanesulfonate

Produit de départ : *N*-[2-(5-Hydroxy-1-benzothiophèn-3-yl)éthyl]-2-méthylpropanamide, obtenu en procédant comme dans la Préparation 1 à partir du *N*-[2-(5-méthoxy-1-benzothiophèn-3-yl)éthyl]-2-méthylpropanamide.

### Préparation 54 : 3-[2-(Heptanoylamino)éthyl]-1H-pyrrolo[2,3-b]pyridin-5-yl trifluorométhanesulfonate

Produit de départ : *N*-[2-(5-Hydroxy-*1H*-pyrrolo[*2,3-b*]pyridin-3-yl)éthyl]heptanamide, obtenu en procédant comme dans la Préparation 1 à partir du *N*-[2-(5 -méthoxy-*1H*-pyrrolo[*2,3-b*]pyridin-3-yl)éthyl]heptanamide.

### Préparation 55 : 3-[2-(Acétylamino)éthyl]-1H-pyrrolo[2,3-b]pyridin-5-yl trifluorométhanesulfonate

Produit de départ : Préparation 12

### Préparation 56 : 3-[4-(Cyclopentylamino)-4-oxobutyl]-1-benzofuran-5-yl trifluorométhanesulfonate

Produit de départ : *N*-Cyclopentyl-4-(5-hydroxy-1-benzofuran-3-yl)butanamide, obtenu en procédant comme dans la Préparation 1 à partir du *N*-cyclopentyl-4-(5-méthoxy-1-benzofuran-3-yl)butanamide.

### Préparation 57 : 3-{2-[(Cyclopropylcarbonyl)amino]éthyl}-1H-pyrrolo-[2,3-b] pyridin-5-yl trifluorométhanesulfonate

Produit de départ : Préparation 10

### Préparation 58 : 3-(2-{[(Allylamino)carbonyl]amino}éthyl)-1-benzothiophèn-5-yl trifluorométhanesulfonate

Produit de départ : *N*-Allyl-*N'*-[2-(5-hydroxy-1-benzothiophèn-3-yl)éthyl]urée, obtenue en procédant comme dans la Préparation 1 à partir de la *N*-allyl-*N*'-[2-(5-Méthoxy-1-benzothiophèn-3-yl)éthyl]urée.

### Préparation 59 : 3-[(Acétylamino)éthyl]-1,4-benzodioxin-6-yl trifluorométhanesulfonate

Produit de départ : Préparation 18

### Préparation 60 : 3-[2-(Isobutyrylamino)éthyl]-1-benzofuran-5-yl trifluorométhanesulfonate

Produit de départ : Préparation 7

### Préparation 61 : 4-{2-[(2,2,2-Trifluoroacétyl)amino]éthyl}-3,4-dihydro-2H-chromèn-6-yl trifluorométhanesulfonate

Produit de départ : 2,2,2-Trifluoro-*N*-[2-(6-hydroxy-3,4-dihydro-*2H*-chromèn-4-yl)éthyl] acétamide, obtenu en procédant comme dans la Préparation 1 à partir du 2,2,2-trifluoro-*N*-[2-(6-Méthoxy-3,4-dihydro-*2H*-chromèn-4-yl) éthyl]acétamide.

### Préparation 62 : 3-(4-Anilino-4-oxobutyl)-1-benzothiophèn-5-yl trifluorométhanesulfonate

Produit de départ : 4-(5-Hydroxy-1-benzothiophèn-3-yl)-*N*-phénylbutanamide, obtenu en procédant comme dans la Préparation 1 à partir du 4-(5-méthoxy-1-benzothiophèn-3-yl)-*N*-phénylbutanamide.

### Préparation 63 : 3-[(Acétylamino)méthyl]-3,4-dihydro-2H-chromèn-6-yl trifluorométhanesulfonate

Produit de départ : *N*-[(6-Hydroxy-3,4-dihydro-2*H*-chromèn-3-yl)méthyl]acétamide, obtenu en procédant comme dans la Préparation 1 à partir du *N*-[(6-méthoxy-3,4-dihydro-*2H*-chromèn-3-yl)méthyl]acétamide.

### Préparation 64 : 3-[2-(Acétylamino)éthyl]-2-[4-(trifluorométhyl)benzyl]-1-benzofuran-5-yl trifluorométhanesulfonate

Produit de départ : *N*-(2-{5-Hydroxy-2-[4-(trifluorométhyl)benzyl]-1-benzofuran-3-yl}éthyl) acétamide, obtenu en procédant comme dans la Préparation 1 à partir du *N*-(2-{5-méthoxy-2-[4-(trifluorométhyl)benzyl]-1-benzofuran-3-yl}éthyl)acétamide.

### Préparation 65 : 3-(2-{[(Méthylamino)carbonyl]amino}éthyl)-1H-indol-5-yl trifluorométhanesulfonate

Produit de départ : *N*-[2-(5-Hydroxy-*1H*-indol-3-yl)éthyl]-*N*'-méthylurée, obtenu en procédant comme dans la Préparation 1 à partir de la *N*-[2-(5-méthoxy-*1H*-indol-3-yl)éthyl]-*N*'-méthylurée.

### Préparation 66 : 4-{2-[(2,2-Diméthylpropanoyl)amino]éthyl}-3,4-dihydro-2H-chromèn-6-yl trifluorométhanesulfonate

Produit de départ : *N*-[2-(6-Hydroxy-3,4-dihydro-*2H*-chromèn-1-yl)éthyl]-2,2-diméthyl-propanamide, obtenu en procédant comme dans la Préparation 1 à partir du *N*-[2-(6-méthoxy-3,4-dihydro-*2H*-chromèn-4-yl)éthyl]-2,2-diméthyl-propanamide.

### Préparation 67 : 3-[2-(Acétylamino)éthyl]-1H-indol-5-yl trifluorométhanesulfonate

Produit de départ : *N*-acétylsérotonine.

### Préparation 68 : 3-{[(Cyclohexylcarbonyl)amino]méthyl]-1,4-benzodioxin-6-yl trifluorométhanesulfonate

Produit de départ : *N*-[(7-Hydroxy-1,4-benzodioxin-2-yl)méthyl]cyclohexane carboxamide, obtenu en procédant comme dans la Préparation 1 à partir du *N*-[(7-méthoxy-1,4-benzodioxin-2-yl)méthyl]cyclohexane carboxamide.

### Préparation 69 : 3-[2-(Acétylamino)éthyl]-2-(3-méthoxybenzyl)-1-benzothiophèn-5-yl trifluorométhanesulfonate

Produit de départ : *N*-{2-[5-Hydroxy-2-(3-méthoxybenzyl)-1-benzothiophèn-3-yl]éthyl} acétamide, obtenu en procédant comme dans la Préparation 1 à partir du *N*- {2-[5-méthoxy-2-(3-méthoxybenzyl)-1-benzothiophèn-3-yl] éthyl}acétamide.

### Préparation 70 : 3-[3-(Acétylamino)propyl]-1-benzofuran-5-yl trifluorométhane sulfonate

Produit de départ : *N*-[3-(5-Hydroxy-1-benzofuran-3-yl)propyl]acétamide, obtenu en procédant comme dans la Préparation 1 à partir du *N*-[3-(5-méthoxy-1-benzofuran-3-yl)propyl]acétamide.

### Préparation 71 : N-{2-[5-Hydroxy-1-(phénylsulfonyl)-1H-indol-3-yl]éthyl}acétamide

### Stade A : N-{2-[5-Méthoxy-1-(phénylsulfonyl)-1H-indol-3-yl]éthyl}acétamide

On dissout 5 g de mélatonine dans 150 ml de dichlorométhane, puis 3.41 g de soude et 0,35 g d'hydrogénosulfate de tétrabutylammonium sont additionnés. Le milieu réactionnel est ensuite refroidi dans un bain de glace et 4.06 ml de chlorure de benzènesulfonyle sont ajoutés goutte à goutte. Après une nuit d'agitation à température ambiante, l'excès de soude et le catalyseur sont filtrés, le solvant est éváporé sous vide et le solide obtenu est recristallisé pour conduire au produit du titre sous la forme de cristaux blancs. Point de fusion : 140-141°C.

### Stade B : N-{2-[5-Hydroxy-1-(phénylsulfonyl) 1H-indol-3-yl]éthyl}acétamide

On dissout 5 g du composé obtenu au stade A dans 100 ml de dichlorométhane. Le milieu réactionnel est ensuite refroidi dans un bain de glace et 3.81 ml de tribromure de bore sont ajoutés goutte à goutte. Après deux heures d'agitation à température ambiante, le milieu réactionnel est versé dans 500 ml d'eau et de glace. Le précipité formé est filtré, lavé à l'eau et séché à l'étuve à 50°C.
Point de fusion : 205-206°C

### Préparation 72 : 3-[2-(Acétylamino)éthyl]-1-benzothien-5-yl trifluorométhanesulfonate

On procède comme dans la Préparation 45 à partir du composé obtenu dans la Préparation 9.

### Example 1: N-(2-{7-[2-({3-[2-(Acétylamino)éthyl]-1-benzofuran-5-yl}oxy)éthoxy]-1-naphtyl}éthyl)acétamide

### Stade A : N-{2-[7-(2-Bromoéthoxy)napht-1-yl]éthyl}acétamide

On dissout le composé obtenu dans la Préparation 1 (0,009 mol) dans 20 ml d'un mélange de diméthylsulfoxyde (6 ml) et de butanone (14 ml). On ajoute 0,027 mol de carbonate de potassium et 0,036 mol de dibromoéthane, puis on chauffe à reflux pendant 48 heures. Le milieu réactionnel est ensuite refroidi et versé dans l'eau. La phase aqueuse est extraite par Et₂O, puis la phase organique est lavée à l'eau jusqu'à neutralité des eaux de lavage, puis séchée sur sulfate de magnésium et évaporée sous pression réduite. Le résidu obtenu est purifié par chromatographie sur gel de silice (éluant : acétone/cyclohexane (2/8)) et recristallisé. Solide blanc.
Point de fusion : 110-111°C.

### Microanalyse élémentaire :

| % | C | H | N |
|---|---|---|---|
| Calculé | 57,15 | 5,40 | 4,17 |
| Trouvé | 57,28 | 5,38 | 3,91 |

### Stade B : N-(2-{7-[2-({3-[2-(Acétylamino)éthyl]-1-benzoluran-5-yl}oxy)éthoxy]-1-naphtyl}éthyl)acétamide

Dans un ballon de 100 ml, on dissout 0,003 mol du composé obtenu dans la Préparation 2 et 0,003 mol du composé obtenu au stade A dans un mélange constitué de 3 ml de diméthylsulfoxyde et 20 ml de butanone. On ajoute 0.009 mol de carbonate de potassium et un cristal d'iodure de potassium puis on chauffe à reflux pendant 12 heures. Le milieu réactionnel est ensuite refroidi et versé dans 100 ml d'eau. Le précipité formé est essoré et recristallisé.

### Exemple 2 : N-(2-{5-[2-({8-[2-(Acétylamino)éthyl]-2-naphtyl}oxy)éthoxy]-1-benzofuran-3-yl}éthyl)cyclopropanecarboxamide

On procède comme dans l'Exemple 1 en remplaçant dans le stade B le composé obtenu dans la Préparation 2 par le composé obtenu dans la Préparation 3.

### Exemple 3 : N-(2-{5-[2-({8-[2-(Acétylamino)éthyl]-2-naphtyl}oxy)éthoxy]-1-benzofuran-3-yl}éthyl)-2-furamide

On procède comme dans l'Exemple 1 en remplaçant au stade B le compose obtenu dans la Préparation 2 par le composé obtenu dans la Préparation 4.

### Exemple 4 : N-(2-{7-[2-([3-[2-(Acétylamino)éthyl]-1-benzofuran-5-yl}thio)éthoxy]-1-naphtyl}éthyl)benzamide

On procède comme dans l'Exemple 1 en remplaçant ;
- dans le stade A, le composé obtenu dans la Préparation 1 par le composé obtenu dans la Préparation 5,
- dans le stade B, le composé obtenu dans la Préparation 2 par le composé obtenu dans la Préparation 36.

### Exemple 5 : N-(2-{7-[2-({3-[2-(Acétylamino)éthyl]-1-benzofuran-5-yl}amino) éthoxy]-1-naphtyl}éthyl)acétamide

On procède comme dans l'Exemple 1 en remplaçant dans le stade B le composé obtenu dans la Préparation 2 par le composé obtenu dans la Préparation 40.

### Exemple 6 : N-(2-{7-[2-({3-[2-(Isobutyrylamino)éthyl]-1-benzofuran-5-yl}oxy) éthoxy]-1-naphtyl}éthyl)-3-buténamide

On procède comme dans l'Exemple 1 en remplaçant :
- dans le stade A, le composé obtenu dans la Préparation 1 par le composé obtenu dans la Préparation 6,
- dans le stade B, le composé obtenu dans la Préparation 2 par le composé obtenu dans la Préparation 7.

### Exemple 7 : N-(2-{7-[2-({3-[2-(Acétylamino)éthyl]-1-benzothiophèn-5-yl}oxy) éthoxy]-1-naphtyl}éthyl)-2-phénylacétamide

On procède comme dans l'Exemple 1 en remplaçant :
- dans le stade A, le composé obtenu dans la Préparation 1 par le composé obtenu dans la Préparation 5,
- dans le stade B, le composé obtenu dans la Préparation 2 par le composé obteno dans la Préparation 9.

### Exemple 8 : N-(2-{5-[2-({8-[2-(Acétylamino)éthyl]-2-naphtyl}oxy)éthoxy]-1H-pyrrolo[2,3-b]pyridin-3-yl}éthyl)cyclopropanecarboxamide

On procède comme dans l'Exemple 1 en remplaçant dans le stade B le produit obtenu dans la Préparation 2 par le composé obtenu dans la Préparation 10.

### Exemple 9 : N-(2-{5-[2-({3-[2-(Acétylamino)éthyl]-3a,7a-dihydro-1-benzofuran-5-yl}oxy)éthoxy]-1-benzothiophèn-3-yl}éthyl)acétamide

On procède comme dans l'Exemple 1 en remplaçant :
- dans le stade A, le composé obtenu dans la Préparation 1 par le composé obtenu dans la Préparation 2,
- dans le stade B, le composé obtenu dans la Préparation 2 par le composé obtenu dans la Préparation 9.

### Exempte 10 : N-(2-{5-[2-({3-[2-(Acétylamino)éthyl]-1H-indol-5-yl}oxy)éthoxyl]-3a,7a-dihydro-1-benzofuran-3-yl}éthyl)-2-furamide

On procède comme dans l'Exemple 1 en remplaçant :
- dans le stade A, le composé obtenu dans la Préparation 1 par le composé obtenu dans la Préparation 4.
- dans le stade B, le composé obtenu dans la Préparation 2 par le composé obtenu dans la Préparation 11.

### Exemple 11 : N-(2-{5-[2-({3-[2-(Acétylamino)éthyl]-1H-pyrrolo[2,3-b]pyridin-5-yl}oxy)éthoxy]-3a,7a-dihydro-1-benzofuran-3-yl}éthyl)-2-méthylpropanamide

On procède comme dans l'Exemple 1 en remplaçant :
- dans le stade A, le composé obtenu dans la Préparation 1 par le composé obtenu dans la Préparation 7.
- dans le stade B, le composé obtenu dans la Préparation 2 par le composé obtenu dans la Préparation 12.

### Exemple 12 : N-(2-{7-[3-({3-[2-(Acétylamino)éthyl]-1-benzothiophèn-5-yl}oxy) propoxy]-1-naphtyl}éthyl)acétamide

### Stade A : N-{2-[7-(3-Hydroxypropyloxy)napht-1-yl]éthyl}acétamide

Dans un ballon de 100 ml, on dissout 0,022 mol du composé obtenu dans la Préparation 1 dans 30 ml de diméthylformamide. On ajoute 0,066 mol de carbonate de potassium et 0.033 mol de 3-bromopropan-1-ol, puis le mélange est chauffé à 80°C pendant 4 heures. Le milieu réactionnel est refroidi et versé dans 100 ml d'une solution d'HCl 1M. La phase aqueuse est extraite 3 fois avec Et₂O puis la phase organique est séchée sur MgSO₄ et évaporée sous pression réduite, Le produit du titre est obtenu après recristallisation. Solide blanc.
Point de fusion : 141-142°C

### Stade B : 3-({8-[2-(Acétylamino)éthyl]-2-naphtyl}oxy)propylméthanesulfonate

Dans un ballon de 250 ml, l'alcool obtenu au stade A est dissous dans 50 ml de dichlorométhane et on ajoute 0.012 mol de triéthylamine. On refroidit dans un bain de glace-sel à -10°C puis 0,012 mol de chlorure de mésyle sont ajoutées goutte à goutte sous agitation magnétique. Le milieu réactionnel est agité à température ambiante pendant 4 heures. Puis on additionne 100 ml d'eau et on poursuit par une extraction au CH₂Cl₂. La phase organique est lavée à l'eau, séchée sur MgSO₄ et évaporée sous pression réduite. L'huile obtenue est purifiée par chromatographie sur gel de silice (éluant : acétone/cyclohexane (2/8)).

### Stade C : N-(2-{7-[3-({3-[-(Acétylamino)éthyl]-1-benzothiophèn-5-yl}oxy) propoxy]-1-naphtyl}éthyl)acétamide

Dans un ballon de 100 ml contenant 30 ml de méthanol, on ajoute par petites portions 0.06 g de sodium. Lorsque le sodium est totalement consommé, on ajoute 0,0033 mol du composé obtenu dans la Préparation 9 et on laisse agiter pendant 20 minutes. Le méthanol est évaporé sous pression réduite et le résidu est repris dans 15 ml de DMF, puis on ajoute 0,0027 mol du composé obtenu au stade B. Le milieu réactionnel est ensuite chauffé à reflux pendant 12 heures puis refroidi et versé dans 100 ml d'eau et 10 ml d'HCl 3M. Après extraction à l'acétate d'éthyle, la phase organique est lavée avec une solution de soude 10 % puis à l'eau. Après séchage sur MgSO₄, évaporation sous pression réduite du solvant, le composé du titre est purifié par chromatographie sur gel de silice.

### Exemple 13 : N-(2-{7-[3-({3-[2-(Butyrylamino)éthyl]-1-benzothiophèn-5-yl}thio) propoxy]-1-naphtyl}éthyl)cyclobutanecarboxamide

On procède comme dans l'Exemple 12 en remplaçant ;
- dans le stade A, le composé obtenu dans la Préparation 1 par le composé obtenu dans la Préparation 13,
- dans le stade C, le composé obtenu dans la Préparation 9 par le composé obtenu dans la Préparation 37.

### Exemple 14: N-[2-[5-({3-[(8-{2-[(2,2,2-Trifluoroacétyl)amino]éthyl}-2-naphtyl)oxy] propyl}amino)-1-benzothiophèn-3-yl]éthyl}pentanamide

On procède comme dans l'Exemple 12 en remplaçant
- dans le stade A, le compose obtenu dans la Préparation 1 par le composé obtenu dans la Préparation 14,
- dans le stade C. le composé obtenu dans la Préparation 9 par le composé obtenu dans la Préparation 41.

### Exemple 15 : N-({6-[3-({8-[2-(Acétylamino)éthyl]-2-naphtyl]oxy)propoxy]-2H-chromèn-3-yl}méthyl)butanamide

On procède comme dans l'Exemple 12 en remplaçant dans le stade C le composé de la Préparation 9 par le composé de la Préparation 15.

### Exemple 16 : N-(2-{5-[3-({3-[(Acétylamino)méthyl]-2H-chromèn-6-yl]oxy)propoxy]-1-benzofuran-3-yl}éthyl)cyclopropanecarboxamide

On procède comme dans l'Exemple 12 en remplaçant :
- dans le stade A, le composé de la Préparation 1 par le composé de la Préparation 16.
- dans le stade C, le composé de la Préparation 9 par le composé de la Préparation 3.

### Exemple 17 : N-[2-[5-(3-{[3-({(Propylamino)carbonyl]amino}méthyl)-1,4-benzodioxin-6-yl]oxy}propoxy)-1H-pyrrolo[2,3-b]pyridin-3-yl]éthyl} acétamide

On procède comme dans l'Exemple 12 en remplaçant :
- dans le stade A, le composé de la Préparation 1 par le composé de la Préparation 17.
- dans le stade C, le composé de la Préparation 9 par le composé de la Préparation 12.

### Exemple 18 : N-({7-[4-({8-[2-(Acétylamino)éthyl]-2-naphtyl}oxy)butoxy]-1,4-benzodioxin-2-yl}méthyl)acétamide

### Stade A : 4-({8-[2-(Acétylamino)éthyl]-2-naphtyl}oxy)butanoate d'éthyle

Dans un ballon de 100 ml, on dissout 0.022 mol du composé obtenu dans la Préparation 1 dans 50 ml d'acétonitrile. On ajoute 0,066 mol de carbonate de potassium et la réaction est agitée à 80°C pendant 30 minutes. 0,033 mol de 1-bromobutyrate d'éthyle sont ensuite ajoutées goutte à goutte et la réaction est agitée 1 heure à 80°C. L'acétonitrile est évaporé sous pression réduite et le résidu solubilisé dans une solution d'HCl 1N. Après extraction à l'acétate d'éthyle, lavage de la phase organique à l'eau, séchage sur MgSO₄ et évaporation sous pression réduite, le composé du titre est purifié par recristallisation. Solide beige.
Point de fusion : 64-66°C

### Stade B: N-{2-[7-(4-Hydroxybutyloxy)napht-1-yl]éthyl}acétamide

Dans un ballon de 250 ml, l'ester obtenu au stade A (0.009 mol) est dissous dans 100 ml d'éther anhydre. 0,009 mol d'hydrure mixte de lithium et d'aluminium sont ajoutées par portions et la réaction est agitée 6 heures à l'ambiante. Le milieu réactionnel est ensuite hydrolysé par quelques gouttes de NaOH 1M et le précipité formé est filtré. Le filtrat est séché sur MgSO₄ et évaporé sous pression réduite. Le résidu obtenu est précipité dans un mélange Et₂O/Ether de pétrole (1/1), essoré ct recristallisé. Solide blanc.
Point de fusion : 82-84°C

### Microanalyse élémentaire :

| % | C | H | N |
|---|---|---|---|
| Calculé | 71,73 | 7,69 | 4,64 |
| Trouvé | 72,00 | 7,58 | 4,45 |

### Stade C : 4-({8-[2-(Acétylamino)éthyl]-2-naphtyl}oxy)butyl méthanesulfonate

On procède comme dans le stade B de l'Exemple 12 à partir du composé obtenu au stade B.

### Stade D : N-({7-[4-({8-[2-(Acétylamino)éthyl]-2-naphtyl}oxy)butoxy]-1,4-benzodioxin-2-yl}méthyl)acétamide

On procède comme dans le stade C de l'Exemple 12 en remplaçant le composé obtenu dans la Préparation 9 par le composé obtenu dans la Préparation 18.

### Exemple 19 : N-{2-[7-(4-{[3-[(Acétylamino)éthyl]-2-(3-méthoxybenzyl)-1-benzofuran-5-yl]amino}butoxy)-1-naphtyl]éthyl}-2-furamide

On procède comme dans l'Exemple 18 en remplaçant dans le stade A le composé de la Préparation 1 par le composé de la Préparation 19, et dans le stade D le composé de la Préparation 9 par le composé de la Préparation 42.

### Exemple 20 : N-({6-[4-({3-[(Acétylamino)éthyl]-2-benzyl-1H-pyrrolo[2,3-b]pyridin-5-yl}oxy)butoxy]-4a,8a-dihydro-2H-chromèn-3-yl}méthyl)butanamide

On procède comme dans l'Exemple 18 en remplaçant dans le stade A le composé de la Préparation 1 par le composé de la Préparation 15, et dans le stade D le composé de la Préparation 9 par le composé de la Préparation 20.

### Exemple 21 : N-(2-{5-[4-({3-[2-(Acétylamino)éthyl]-2-[4-(trifluorométhyl)benzyl]-1-benzothiophèn-5-yl]thio)butoxy]-1H-pyrrolo[2,3-b]pyridin-3-yl}éthyl) cyclopropanecarboxamide

On procède comme dans l'Exemple 18 en remplaçant dans le stade A le composé de la Préparation 1 par le composé de la Préparation 10, et dans le stade D le composé de la Préparation 9 par le composé de la Préparation 38.

### Exemple 22 : N-(2-{5-[4-({3-[(Acétylamino)méthyl]-4a,8a-dihydro-2H-chromèn-6-yl}oxy)butoxy]-1-benzothiophèn-3-yl}éthyl)cyclobexanecarboxamide

On procède comme dans l'Exemple 18 en remplaçant dans le stade A le composé de la Préparation 1 par le composé de la Préparation 16, et dans le stade D le composé de la Préparation 9 par le composé de la Préparation 21.

### Exemple 23 : 2,2,2-Trifluoro-N-(2-{5-[4-({3-[2-(hexylamino)-2-oxoéthyl]-3a,7a-dihydro-1-benzofuran-5-yl}oxy)butoxy]-1-benzothiophèn-3-yl}éthyl) acétamide

On procède comme dans l'Exemple 18 en remplaçant dans le stade A le composé de la Préparation 1 par le composé de la Préparation 22, et dans le stade D le composé de la Préparation 9 par le composé de la Préparation 23.

### Exemple 24 : N-(2-{7-[4-({4-[2-(Acétylamino)éthyl]-3,4-dihydro-2H-chromèn-6-yl} oxy)butoxy]-1,2,3,4-tétrahydro-1-naphtalényl}éthyl)acétamide

On procède comme dans l'Exemple 18 en remplaçant dans le stade A le composé de la Préparation 1 par le composé de la Préparation 24, et dans le stade D le composé de la Préparation 9 par le composé de la Préparation 25.

### Exemple 25 : N-{2-[5-({4-[(8-{2-[(Cyclopropylcarbonyl)amino]éthyl}-5,6,7,8-tétrahydro-2-naphtalényl)oxy]butyl}amino)-1-benzofuran-3-yl]éthyl}-2-furamide

On procède comme dans l'Exemple 18 en remplaçant dans le stade A le composé de la Préparation 1 par le composé de la Préparation 26, et dans le stade D le composé de la Préparation 9 par le composé de la Préparation 43.

### Exemple 26 : N-(2-{5-[4-({8-[2-(Heptanoylamino)éthyl]-2-naphtyl}oxy)butoxy]-1H-indol-3-yl}éthyl)cyclobutanecarboxamide

On procède comme dans l'Exemple 18 en remplaçant dans le stade A le composé de la Préparation 1 par le composé de la Préparation 27, et dans le stade D le composé de la Préparation 9 par le composé de la Préparation 28.

### Exemple 27 : N-[2-(5-{[6-({8-[2-(Acétylamino)éthyl-2-naphtyl}oxy)hexyl]oxy}-1H-pyrrolo[2,3-b]pyridin-3-yl)éthyl]acétamide

### Stade A : N-(2-{7-[(6-Hydroxyhexyl)oxy]-1-naphtyl}èthyl)acétamide

On procède comme dans le stade A de l'Exemple 12 en remplaçant le 3-bromopropan-1-ol par le 6-bromohexan-1-ol. Solide blanc.
Point de fusion : 58-61°C

### Microanalyse élémentaire :

| % | C | H | N |
|---|---|---|---|
| Calculé | 72,91 | 8,41 | 4,25 |
| Trouvé | 73,22 | 8,17 | 4,02 |

### Stade B : 6-({8-[2-(Acétylamino)éthyl]-2-naphtyl}oxy)hexyl méthanesulfonate

On procède comme dans le stade B de l'Exemple 12. Solide blanc.
Point de fusion : 66-67°C

### Stade C : N-{2-(5-{[6-({8-[2-(Acétylamino)éthyl] 2-naphtyl}oxy)hexyl]oxy}-1H-pyrrolo[2,3-b]pyridin-3-yl)éthyl}acétamide

On procède comme dans le stade C de l'Exemple 12 en remplaçant le composé obtenu dans la Préparation 9 par le composé obtenu dans la Préparation 12.

### Exemple 28 : 4-(7-{[6-({3-[(Acétylamino)méthyl]-2H-chromèn-6-yl}oxy)hexyl]oxy}-1-naphtyl)-N-isopropylbutanamide

On procède comme dans l'Exemple 27 en remplaçant dans le stade A le composé obtenu dans la Préparation 1 par le composé obtenu dans la Préparation 29, et dans le stade C le composé obtenu dans la Préparation 9 par le composé obtenu dans la Préparation 16.

### Exemple 29 : N-{[7-({6-[(3-{2-[(Anilinocarbonyl)amino]éthyl}-1-benzofuran-5-yl) oxy]hexyl}oxy)-1,4-benzodioxin-2-yl]méthyl}acétamide

On procède comme dans l'Exemple 27 en remplaçant dans le stade A le composé de la Préparation 1 par le composé obtenu dans la Préparation 30, et dans le stade C le composé de la Préparation 9 par le composé obtenu dans la Préparation 18.

### Exemple 30 : N-[2-(7-{[6-({3-[2-(Benzylamino)-2-oxoéthyl]-1-benzothiophèn-5-yl}oxy)hexyl]thio}-1,2,3,4-tétrahydro-1-naphtalényl)éthyl] cyclopropanecarboxamide

On procède comme dans l'Exemple 27 en remplaçant :
- dans le stade A, le composé de la Préparation 1 par le composé de la Préparation 31.
- dans le stade C, le composé de la Préparation 9 par le composé de la Préparation 26.

### Exemple 31 : N-[2-(5-{[6-({3-[3-(Méthylamino)-3-oxopropyl]-1-benzofuran-5-yl}oxy)hexyl]oxy}-1H-indèn-3-yl)éthyl]pentanamide

On procède comme dans l'Exemple 17 en remplaçant dans le stade A le composè de la Préparation 1 par le composé de la Préparation 32, et dans le stade C le composé de la Préparation 9 par le composé de la Préparation 33.

### Exemple 32 : N-Cyclopropyl-N'-(2-{5-[(6-{[3-(2-([(méthylamino)carbonyl]amino] éthyl)-1H-pyrrolo[2,3-b]pyridin-5-yl]oxy)hexyl)amino]-1-benzofuran-3-yl}éthyl)urée

On procède comme dans l'Exemple 27 en remplaçant dans le stade A le composé de la Préparation 1 par le composé de la Préparation 34, et dans le stade C le composé de la Préparation 9 par le composé de la Préparation 44.

### Exemple 33 : N-[2-(7-{[6-((3-[4-(Méthylamino)-4-oxobutyl]-1H-indol-5-yl}oxy) hexyl]oxy}-1-naphtyl)éthyl]-3-buténamide

On procède comme dans l'Exemple 27 en remplaçant :
- dans le stade A, le composé de la Préparation 1 par le composé de la Préparation 6.
- dans le stade C, le composé de la Préparation 9 par le composé de la Préparation 35.

### Exemple 34 : N-[2-(7-{3-[2-(Acétylamino)éthyl]-1-benzofuran-5-yl}-1-naphtyl)éthyl] acétamide

Sous azote, 2,76 mmoles du composé obtenu dans la Préparation 45, 2,76 mmoles du composé obtenu dans la Préparation 46, 1,94 mmoles de dichlorobis (triphénylphosphine) nickel, 3,87 mmoles de triphénylphosphine et 8,30 mmoles de zine sont mis en suspension dans 20 ml de DMF sec. Après chauffage 48 heures à 120°C sous azote, le milieu réactionnel est concentré avant de partager le résidu obtenu entre CH₂Cl₂ et NaHCO₃ 1M. La phase organique est ensuite séchée sur Na₂SO₄ et concentrée sous vide. Le composé du titre est séparé par chromatographie sur gel de silice.

Dans les Exemples 35 à 48, on procède comme dans l'Exemple 34 à partir des Préparations appropriées.

### Exemple 35 : N-(2-{5-[8-(2-{[(Méthylamino)carbonyl]amino}éthyl)-2-naphtyl]-1-benzothiophèn-3-yl}éthyl)cyclopropanecarboxamide

Produits de départ : Préparations 47 et 48

### Exemple 36 : N-{2-[5-(3-{2-[(Anilinocarbonyl)amino]éthyl}-1-benzofuran-5-yl)-1-benzothiophèn-3-yl]éthyl}-2-furamide

Produits de départ : Préparations 49 et 50

### Exemple 37 : 2-(5-{3-[2-(Acétylamino)éthyl]-1-benzofuran-5-yl)-1H-indol-3-yl)-N-benzylacétamide

Produits de départ : Préparations 45 et 51

### Exemple 38 : N-[3-(5-{3-[2-(Isobutyrylamino)éthyl]-1-benzothiophèn-5-yl}-1H-indol-3-yl)propyl]benzamide

Produits de départ : Préparations 52 et 53

### Exemple 39 : N-[3-(5-{8-[2-(Acétylamino)éthyl]-2-naphtyl}-1H-pyrrolo[2,3-b] pyridin-3-yl)propyl]heptanamide

Produits de départ : Préparations 46 et 54

### Exemple 40 : 4-(5-{3-[3-(Acétylamino)éthyl]-1H-pyrrolo[2,3-b]pyridin-5-yl}-1-benzofuran-3-yl)-N-cyclopentylbutanamide

Produits de départ : Préparations 55 et 56

### Exemple 41 : N-(2-{5-[3-(2-{[(Allylamino)carbonyl]amino}éthyl)-1-benzothiophèn-5-yl]-1H-pyrrolo[2,3-b]pyridin-3-yl)cyclopropanecarboxamide

Produits de départ : Préparations 57 et 58

### Exemple 42 : N-[2-(5-{3-[(Acétylamino)méthyl]-1,4-benzodioxin-6-yl}-1H-pyrrolo[2,3-b]pyridin-3-yl)éthyl]cyclopropanecarboxamide

Produits de départ : Préparations 57 et 59

### Exemple 43 : 2-Méthyl-N-{2-[5-(4-{2-[(2,2,2-trifluoroacétyl)amino]éthyl}-3,4-dihydro-2H-chromèn-6-yl)-1-benzofuran-3-yl]éthyl}propanamide

Produits de départ : Préparations 60 et 61

### Exemple 44 : 4-(5-{3-[(Acétylamino)méthyl]-3,4-dihydro-2H-chromèn-6-yl}-1-benzothiophèn-3-yl)-N-phénylbatanamide

Produits de départ : Préparations 62 et 63

### Exemple 45 : N-(2-{5-{8-[2-(Acétylamino)éthyl]-2-naphthyl}-2-[4-(trifluorométhyl) benzyl]-1-benzofuran-3-yl}éthyl)acétamide

Produits de départ : Préparations 64 et 46

### Exemple 46 : 2,2-Diméthyl-N-(2-{6-[3-(2-{[(méthylamino)carbonyl]amino}éthyl]-1H-indol-5-yl]-3,4-dihydro-2H-chromèn-4-yl}éthyl)propanamide

Produits de départ : Préparations 65 et 66

### Exemple 47 : N-[(7-{3-[2-(Acétylamino)éthyl]-1H-indol-5-yl}-1,4-benzodioxin-2-yl) méthyl]cyclohexanecarboxamide

Produits de départ : Préparations 67 et 68

### Exemple 48 : N-(3-{5-[3-[2-(Acétylamino)éthyl]-2-(3-méthoxybenzyl)-1-benzothiophèn-5-yl]-1-benzofuran-3-yl}propyl)acétamide

Produits de départ : Préparations 69 et 70

### Exemple 49 : N-(2-{7-[4-({3-[2-(Acétylamino)éthyl]-1-benzefuran-5-yl}oxy)butoxy]-1-naphtyl}éthyl)acétamide

### Stade A : N-{2-[7-(4-Bromobutoxy)-1-naphtyl]éthyl]acétamide

Dans un ballon de 100 ml, 10 mmol du composé obtenu dans la préparation 1 sont dissoutes dans 50 ml d'acétonitrile. On ajoute 30 mmol de carbonate de potassium et on laisse sous agitation magnétique à reflux 30 minutes puis 10 mmol de 1,4-dibromobutane sont ajoutées. Après 12 heures à reflux, l'acétonitrile est évaporé sous vide et le résidu obtenu est repris par une solution de soude 1M. Le précipité obtenu est filtré et recristallisé pour conduire au produit du titre.

### Stade B : N-(2-{7-[4-({3-[2-(Acétylamino)éthyl]-1-benzofuran-5-yl}oxy)butoxy]-1-naphtyl}éthyl)acétamide

Dans un ballon de 100 ml contenant 30 ml de méthanol, on ajoute par petites portions le sodium (0,07 g; 0,0030 at.g.). Lorsque le sodium est totalement consommé, on ajoute 3,6 mmol du composé obtenu dans la préparation 2. Après 20 minutes d'agitation, le méthanol est évaporé sous pression réduite et le résidu est repris par 15 ml de DMF. On ajoute ensuite 3 mmol du composé obtenu au stade A et on laisse à reflux pendant 12 heures. Le milieu réactionnel est refroidi et on verse sur le mélange 100 ml d'eau et 10 ml d'acide chlorhydrique 3M. On extrait 2 fois la phase aqueuse par de l'acétate d'éthyle et la phase organique est lavée avec une solution de soude à 10% puis à l'eau. Le solide obtenu est recristallisé dans l'acétonitrile pour conduire au produit du titre.
Point de fusion : 160-162°C

### Exemple 50 : N-{2-[5-[4-({8-[2-(Acétylamino)éthyl]-2-naphtyl}oxy)butoxy]-1-(phénylsulfonyl)-1H-indol-3-yl]éthyl]acétamide

Dans un ballon de 100 ml, on dissout 10 mmol du composé obtenu dans la préparation 71 dans 50 ml d'acétonitrile, puis 4,17 g de carbonate de potassium sont additionnés et le milieu est laissé sous agitation magnétique à reflux 30 minutes. 10 mmol du composé obtenu au stade A de l'Exemple 49 sont ensuite additionnés et on chauffe à reflux pendant 12 heures. L'acétonitrile est évaporé sous vide et le résidu obtenu est repris par une solution aqueuse de soude 1M. Le précipité obtenu est filtré et recrisyallisédans l'alcool à 95°.
Point de fusion : 135-137°C

### Exemple 51 : N-(2-{7-[4-({8-[2-(Acétylamino)éthyl]-2-naphthyl}oxy)butoxy]-1,2,3,4-tétrahydro-1-naphtalényl}éthyl)acétamide

On procède comme dans l'Exemple 49 en remplaçant au stade B le produit obtenu dans la préparation 2 par le produit obtenu dans la préparation 25.
Recristallisation dans l'acétonitrile.
Point de fusion : 63-65°C

### Exemple 52 : N-(2-{5-[4-({3-[2-(Acétylamino)éthyl]-1-benzofuran-5-yl}oxy)butoxy]-1H-indol-3-yl}éthyl)acétamide

### Stade A : 4-({3-[2-(Acétylamino)éthyl]-1H-indol-5-yl]oxy)butonoate d'éthyle

On dissout 5,9 g du composé obtenu dans la préparation 11 dans 100 ml d'acétonitrile, puis 11,22 g de carbonate de potassium et 5.81 ml de 4-bromobutanoate d'éthyle sout ajoutés. Après une nuit à reflux, on filtre le carbonate de potassium, l'acétonitrile est évaporé et le résidu repris dans 100 ml d'eau. On extrait par 3 fois 50 ml d'acétate d'éthyle puis la phase organique est lavée à l'eau jusqu'à pH neutre, séchée sur sulfate de magnésium et évaporée sous vide. L'huile obtenue précipite dans l'éther isopropylique.
Point de fusion : 107-108°C

### Stade B : N-{2-[5-(4-Hydroxybutoxy)-1H-indol-3-yl]éthyl}acétamide

A une suspension de 1,42 g d'hydrure d'aluminium et de lithium dans 50 ml de THF anhydre refroidi dans un bain de glace, on ajoute goutte à goutte une solution de 6,2 g du composé obtenu au stade A dans 50 ml de THF anhydre. Après 30 minutes d'agitation à température ambiante, on ajoute goutte à goutte une solution de soude à 5% jusqu'à l'arrêt du dégagement gazeux. Le précipité formé est filtré, la phase organique est évaporée et le résidu repris dans 70 ml d'acétate d'éthyle. La phase organique est lavée à l'eau jusqu'à neutralité, séchée sur sulfate de magnésium et évaporée sous vide pour conduire au produit du titre sous la forme d'une huile.

### Stade C: N-{2-[5-(4-Bromobutoxy)-1H-indol-3-yl]éthyl}acétamide

On dissout 3,92 g du composé obtenu au stade B dans 50 ml d'acétonitrile, puis 5,31 g de triphénylphosphine ct 6,71 g de tétrabromure de carbone sont additionnés sous agitation. Après une nuit à l'ambiante, l'acétonitrile est évaporé sous vide et le résidu obtenu est purifié par chromatographie sur colonne de gel de silice (éluant : dichlorométhane-méthanol 96/4).
Huile.

### Stade D : N-(2-{5-[4-({3-[2-(Acétylamino)éthyl]-1-benzofuran-5-yl}oxy)butoxy]-1H-indol-3-yl}éthyl)acétamide

On dissout 0,72 g du composé obtenu au stade C dans 20 ml d'acétonitrile, puis 0,57 g de carbonate de potassium et 0,30 g du composé obtenu dans la préparation 2 sont additionnés. Après une nuit à reflux, le milieu réactionnel est versé sur 200 ml d'eau et de glace. Le précipité obtenu est filtré, lavé à l'éther, séché et recristallisé pour conduire au produit du titre sous la forme d'une poudre blanche.
Point de fusion : 164-166°C

### Exemple 53 : N-(2-{7-[4-({3-[2-(Acétylamino)éthyl]-1-benzothien-5-yl}oxy)butoxy]-1-naphtyl}éthyl)acétamide

On procède comme dans l'Exemple 49 en remplaçant au stade B le produit obtenu dans la préparation 2 par le produit obtenu dans la préparation 9.
Recristallisation dans acétonitrile/méthanol (2/1).
Point de fusion : 169-170°C

### Exemple 54 : N-(2-{5-[4-({3-[2-(Acétylamino)éthyl]-1-benzothien-5-yl}oxy)butoxy]-1H-indol-3-yl}éthyl)acétamide

On procède comme dans l'Exemple 49 en remplaçant au stade A le produit obtenu dans la Préparation 1 par le produit obtenu dans la Préparation 9 et au stade B le produit obtenu dans la Préparation 2 par le produit obtenu dans la Préparation 11.

### Exemple 55 : N-(2-{5-[4-({3-[2-(Acétylamino)éthyl]-1-benzothien-5-yl]oxy)butoxy]-1H-pyrrolo[2,3-b]pyridin-3-yl)éthyl)acétamide

On procède comme dans l'Exemple 49 en remplaçant au stade A le produit obtenu dans la Préparation 1 par le produit obtenu dans la Préparation 9 et au stade B le produit obtenu dans la Préparation 2 par le produit obtenu dans la Préparation 12.

### Exemple 56 : N-(2-{5-[4-({3-[2-(Acétylamino)éthyl]-1-benzofuran-5-yl}oxy)butoxy]-1H-pyrrolo[2,3-b]pyridin-3-yl}éthyl)acétamide

On procède comme dans l'Exemple 49 en remplaçant au stade A le produit obtenu dans la Préparation 1 par le produit obtenu dans la Préparation 2 et au stade B le produit obtenu dans la Préparation 2 par le produit obtenu dans la Préparation 12.

### Exemple 57 : N-(2-{5-[4-({3-[2-(Acétylamino)éthyl]-1H-indol-5-yl}oxy)butoxy]-1H-pyrrolo[2,3-b]pyridin-3-yl}éthyl)acétamide

On procède comme dans l'Exemple 49 en remplaçant au stade A le produit obtenu dans la Préparation 1 par le produit obtenu dans la Préparation 11 et au stade B le produit obtenu dans la Préparation 2 par le produit obtenu dans In Préparation 12.

Les Exemples 58 à 64 sont obtenus en procédant comme dans l'Exemple 34 à partir des Préparations appropriées.

### Exemple 58 : N-[2-(7-{3-[2-(Acétylamino)éthyl]-1-benzothien-5-yl}-1-naphtyl)éthyl]acétamide

Produits de départs : Préparations 46 et 72.

### Exemple 59 : N-[2-(5-{8-[2-(Acétylamino)éthyl]-2-naphtyl}-1H-pyrrolo[2,3-b] pyridin-3-yl)éthyl]acétamide

Produits de départs : Préparations 46 et 55.

### Exemple 60 : N-[2-(5-{3-[2-(Acétylamino)éthyl]-1-benzofuran-5-yl}-1-benzothien-3-yl)éthyl]acétamide

Produits de départs : Préparations 72 et 45.

### Exemple 61 : N-[2-(5-{3-[2-(Acétylamino)éthyl]-1-benzofuran-5-yl}-1H-indol-3-yl)éthyl]acétamide

Produits de départs : Préparations 67 et 45.

### Exemple 62 : N-[2-(5-{3-[2-(Acétylamino)éthyl]-1-benzofuran-5-yl)-1H-pyrrolo [2,3-b]pyridin-3-yl)éthyl]acétamide

Produits de départs : Préparations 55 et 45.

### Exemple 63 : N-[2-(5-{3-[2-(Acétylamino)éthyl]-1H-indol-5-yl]-1H-pyrrolo [2,3-b]pyridin-3-yl)éthyl]acétamide

Produits de départs : Préparations 55 et 67.

### Exemple 64 : N-[2-(5-{3-[2-(Acétylamino)éthyl]-1-benzothien-5-yl)-1H-pyrrolo [2,3-b]pyridin-3-yl)éthyl]acétamide

Produits de départs : Préparations 55 et 72.

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la *pars tuberalis* de mouton. La *pars tuberalis* de l'adénohypophyse est en effet caractérisée, chez les mammifères, par une haute densité en récepteurs de la mélatonine (Journal of Neuroendocrinology, 1, pp. 1-4. 1989).

### Protocole

1) Les membranes de *pars tuberalis* de mouton sont préparées et utilisées comme tissu cible duns des expériences de saturation pour déterminer les capacités et affinités de liaison pour la 2-[¹²⁵I]-iodomélatonine.
2) Les membranes de *pars tuberalis* de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

II apparaît que les composés de l'invention possèdent une puissante affinité pour les récepteurs de la mélatonine.

### EXEMPLE C : Etude de liaison aux récepteurs mt₁ et MT₂ de la mélatonine

Les expériences de liaison aux récepteurs mt₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention attestent d'une liaison pour l'un ou l'autre des sous-types réceptoriels mt₁ ou MT₂, ces valeurs étant ≤ 10 µM.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 12). Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12:12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg de *N*-(2-{7-[4-({3-[2-(acétylamino)éthyl]-1-benzofuran-5yl}oxy)butoxy]-1-naphtyl}éthyl)acétamide (Exemple 49) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) :
A-G₁-Cy-G₂-Cy'-G₃-B (I)
dans laquelle :
◆ A représente un groupement de formule 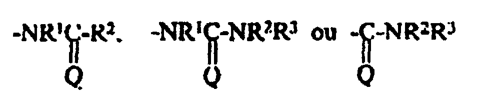 dans lesquelles:
- Q représente un atome de soufre ou d'oxygène,
- R¹, R², R³, identiques ou différents, représentent un atome d'hydrogène ou un groupement Rₐ (où Rₐ représente un groupement alkyle (C₁-C₆) linéaire ou ramifié non substitué ou substitué, alkényle (C₂-C₆) linéaire ou ramifié non substitué ou substitué, alkynyle (C₂-C₆) linéaire ou ramifié non substitué ou substitué, cycloalkyle (C₃-C₈) non substitué ou substitué, cycloalkylalkyle (C₃-C₈) linéaire ou ramifié non substitué ou substitué, polyhalogénoalkyle (C₁-C₆) linéaire ou ramifié, aryle, arylalkyle (C₁-C₆) linéaire ou ramifié, arylalkényle (C₂-C₆) linéaire ou ramifié, hétéroaryle, hétéroarylalkyle (C₁-C₆) linéaire ou ramifié ou hétéroarylalkényle (C₂-C₆) linéaire ou ramifié),
ou les groupements R² et R³ peuvent également former, avec l'atome d'azote qui les porte un groupement choisi parmi piperazinyle, piperidinyle, ou pyrrolidinyle,
◆ B représente un groupement de formule 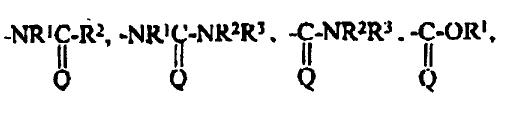 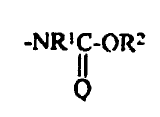 ou -NR²R³ dans lesquelles Q, R¹, R² et R³ sont tels que définis précédemment.
◆ G₁ et G₃, identiques ou différents, représentent une chaîne alkylène contenant de 1 à 4 carbones linéaire ou ramifiée éventuellement substituée par un ou plusieurs groupements, identiques ou différents, choisis parmi hydroxy, carboxy, formyle, Rₐ, ORₐ, COORₐ ou CORₐ (où Rₐ est tel que défini précédemment).
◆ Cy et Cy', différents, représentent
- une structure cyclique de formule (II): 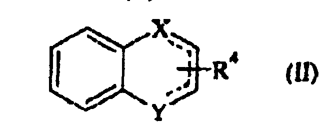 dans laquelle :
* X et Y, identiques ou différents, représentent un atome de soufre, d'oxygène ou de carbone, ou un groupement CH ou CH₂.
* R⁴ représente un atome d'hydrogène ou d'halogène ou un groupement CF₃, hydroxy, carboxy, formyle, amino, NHRₐ, NRₐR¹ₐ, NHCORₐ, CONHRₐ, Rₐ, ORₐ, CORₐ ou COORₐ, (où Rₐ est tel que défini précédemment et R¹ₐ peut prendre toutes les valeurs de Rₐ),
* la représentation ---- signifie que les liaisons sont simples ou doubles, étant entendu que la valence des atomes est respectée.
où G₂ substitue le cycle benzénique, et G₁ substitue le cycle contenant X et Y dans le cas de Cy
et G₂ substitue le cycle benzénique et G₃ substitue le cycle contenant X et Y dans le cas de Cy'.
- ou une structure cyclique de formule (III) : 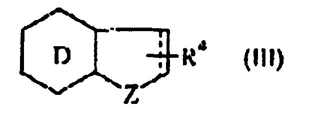 dans laquelle :
* Z représente un atome de soufre ou d'oxygène, ou un groupement CH₂, NH, NSO₂Ph ou NRₐ (où Rₐ est tel que défini précédemment),
* D représente un cycle benzénique ou pyridinique.
* R⁴ est tel que défini précédemment.
* la représentation ---- signifie que la liaison est simple ou double, étant entendu que la valence des atomes est respectée,
où G₂ substitue le cycle D, et G₁ substitue le cycle contenant Z dans le cas de Cy
et G₂ substitue le cycle D et G₃ substitue le cycle contenant Z dans le cas de Cy',
les deux cycles Cy et Cy' des composés de formule (I), différents, étant représentés tous les deux par une structure de formule (II), tous les deux par une structure de formule (III), ou l'un par une structure de formule (II) et l'autre par une structure de formule (III),
• G₇ représente une chaîne de formule (IV) : 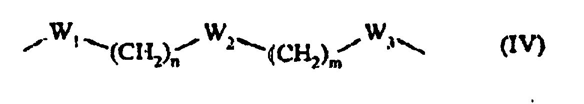 dans laquelle :
- W₁, W₂ et W₃, identiques ou différents, représentent une liaison, un atome d'oxygène ou de soufre, ou un groupement CH₂, CHRₐ, NH ou NRₐ (où Rₐ est tel que défini précédemment).
- n représente un entier tel que 0 ≤ n ≤ 6.
- m représente un entier tel que 0 ≤ m ≤ 6,
étant entendu que l'on ne peut pas avoir deux hétéroatomes consécutifs et que la chaîne de formule (IV) ainsi définie peut comporter une ou plusieurs insaturations,
étant entendu que :
- par « aryle », on entend les groupements naphtyle, phényle et biphényle,
- par « hétéroaryle », on entend tout groupement mono ou bicyclique, saturé ou insaturé contenant 5 à 10 chaînons et contenant 1 à 3 hétéroatomes choisis parmi azote, soufre ou oxygène,
les groupements « aryles » et « hétéroaryles » pouvant être substitués par un ou plusieurs radicaux, identiques ou différents, choisis parmi hydroxy, carboxy, alkoxy (C₁-C₆) linéaire ou ramifié, alkyle (C₁-C₆) linéaire ou ramifié, polyhalogénoalkyle(C₁-C₆) linéaire ou ramifié, formyle, cyano, nitro, amino, alkylamino (C₁-C₆) linéaire ou ramifié, dialkylamino (C₁-C₆) linéaire ou ramifié ou atomes d'halogène,
- le terme « substitué » affecté aux expressions « alkyle », « alkényle », et « alkynyle » signifie que ces groupements sont substitués par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié ou atomes d'halogène,
- le terme « substitué » affecté aux expressions « cycloalkyle » et « cycloalkylalkyle » signifie que la partie cyclique de ces groupements est substituée par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié ou atomes d'halogène,
leurs énantiomères et diastéréoisomères, ainsi que leurs sels d'addition à un acide ou une base pharmaceutiquement acceptable.

2. Composés de formule (I) selon la revendication 1 pour lesquels Cy et Cy', différents, représentent une structure cyclique de formule (II), leurs énantiomères et diastéréoisomères, ainsi que leurs sels d'addition à un acide ou une base pharmaceutiquement acceptable.

3. Composés de formule (I) selon In revendication 1 pour lesquels Cy et Cy', différents, représentent une structure cyclique de formule (III), leurs énantiomères et diastéréoisomères, ainsi que leurs sels d'addition à un acide ou une base pharmaceutiquement acceptable.

4. Composés de formule (I) selon la revendication 1 pour lesquels Cy représente une structure cyclique de formule (II) et Cy' représente une structure cyclique de formule (III). leurs énantiomères et diastéréoisomères, ainsi que leurs sels d'addition à un acide ou une base pharmaceutiquement acceptable.

5. Composés de formule (I) selon la revendication 1 pour lesquels G₂ représente une liaison simple, leurs énantiomères et diastéréoisomères, ainsi que leurs sels d'addition à un acide ou une base pharmaceutiquement acceptable.

6. Composés de formule (I) selon la revendication 1 pour lesquels G₂ représente un groupement -W₄-(CH₂)ₚ-W'₄- dans lequel W₄ et W'₄, identiques ou différents, représentent un atome d'oxygène ou de soufre ou un groupement NH ou NRₐ, et p représente un entier tel que 1 ≤ p ≤ 12, leurs énantiomères et diastéréoisomères, ainsi que leurs sels d'addition à un acide ou une base pharmaceutiquement acceptable.

7. Composés de formule (I) selon la revendication 1 pour lesquels G₂ représente un groupement -O-(CH₂)ₚ-O- dans lequel p représente un entier tel que 1 ≤ p ≤ 12, leurs énantiomères et diastéréoisomères, ainsi que leurs sels d'addition à un acide ou une base pharmaceutiquement acceptable.

8. Composés de formule (I) selon la revendication 1 pour lesquels A et B, identiques ou différents, représentent un groupement NR¹COR² ou CONR²R³, leurs énantiomères et diastéréoisomères, ainsi que leurs sels d'addition à un acide ou une base pharmaceutiquement acceptable.

9. Composés de formule (I) selon la revendication 1 qui sont le *N*-(2-{7-[2-({3-[2-(acétylamino)éthyl]-1-benzofuran-5-yl}oxy)éthoxy]-1-naphtyl}éthyl)acétamide, le *N*-(2-{5-[2-( {8-[2-(acétylamino)éthyl]-2-naphtyl}oxy)éthoxy]-1-benzofuran-3-yl}éthyl)-2-furamide, le *N*-(2-{5-[2-({8-[2-(acétylamino)éthyl)-2-naphtyl}oxy)éthoxy]-*1H*-pyrrolo [*2,3-b*]pyridin-3-yl}éthyl)cyclopropanecarboxamide, le *N*-(2- {7-[3-({3-[2-(acétylamino) éthyl]-1-benzothiophèn-5-yl}oxy)propoxy]-1-naphtyl}éthyl)acétamide, le *N*-[2-(5-{[6-({8-[2-(acétylamino)éthyl]-2-naphtyl}oxy)hexyl]oxy}-*1H*-pyrrolo[*2,3*-*b*]pyridin-3-yl)éthyl] acétamide, le *N*-(2-{5-[4-({3-[2-(acétylamino)éthyl]-1-benzothien-5-yl}oxy)butoxy]-1*H*-indol-3-yl}éthyl)acétamide, le *N*-(2- {5-[4-({3-[2-(acétylamino)éthyl]-1-benzothien-5-yl}oxy)butoxy]-1*H*-pyrrolo[2,3-b]pyridin-3-yl}éthyl)acétamide, le *N*-(2-{5-[4-({3-[2-(acétylamino)éthyl]-1-benzofuran-5-yl}oxy)butoxy]-1*H*-pyrrolo[2,3-*b*]pyridin-3-yl}éthyl) acétamide, le *N*-(2-{5-[4-({3-[2-(acétylamino)éthyl]-1*H*-indol-5-yl}oxy)butoxy]-1*H*-pyrrolo[2,3-*b*]pyridin-3-yl}éthyl)acétamide, ainsi que leurs sels d'addition à un acide ou une base pharmaceutiquement acceptable.

10. Composés de formule (1) selon la revendication 1 qui sont le *N*-(2-{7-[4-({3 [2-(acétylamino)éthyl]-1-benzofuran-5-yl}oxy)butoxy]-1-naphtyl}éthyl)acétamide, le *N*-[2-[5-[4-({8-[2-(acétylamino)éthyl]-2-naphtyl}oxy)butoxy]-1-(phénylsulfonyl)-1*H*-indol-3-yl]éthyl}acétamide, le *N*-(2-{7-[4-({8-[2-(acétylamino)éthyl]-2-naphthyl}oxy)butoxy]-1,2, 3,4-tétrahydro-1-naphtalényl}éthyl)acétamide, le *N*-(2-{5-[4-({3-[2-(acétylamino)éthyl]-1-benzofuran-5-yl}oxy)butoxy]-1*H*-indol-3-yl}éthyl)acétamide, le *N*-(2-{7-[4-({3-[2-(acétyl-amino)éthyl]-1-benzothien-5-yl}oxy)butoxy]-1-naphtyl}éthyl)acétamide, ainsi que leurs sels d'addition à un acide ou une base pharmaceutiquement acceptable.

11. Composés de formule (1) selon la revendication 1 qui sont le *N*-[2-(7-{3-[2-(acétylamino)éthyl]-1-benzofuran-5-yl}-1-naphtyl)éthyl]acétamide, le *N*-[3-(5-{8-[2-(acétylamino)éthyl]-2-naphtyl}-*1H*-pyrrolo[*2,3-b*]pyridin-3-yl)propyl]heptanamide, le *N*-[2-(7-{3-[2-(acétylamino)éthyl]-1-benzothien-5-yl}-1-naphtyl)éthyl]acétamide, le *N*-[2-(5-{8-[2-(acétylamino)éthyl]-2-naphtyl}-1*H*-pyrrolo[2,3-*b*]pyridin-3-yl)éthyl]acétamide, le *N*-[2-(5-{3-[2-(acétylamino)éthyl]-1-benzofuran-5-yl}-1-benzothien-3-yl)éthyl]acétamide, le *N*-[2-(5-{3-[2-(acétylamino)éthyl]-1-benzofuran-5-yl}-1*H*-indol-3-yl)éthyl]acétamide, le *N*-[2-(5-{3-[2-(acétylamino)éthyl]-1-benzofuran-5-yl}-1*H*-pyrrolo[2,3-*b*]pyridin-3-yl)éthyl] acétamide, le *N*-[2-(5-{3-[2-(acétylamino)éthyl]-1*H*-indol-5-yl}-1*H*-pyrrolo[2,3-*b*]pyridin-3-yl)éthyl]acétamide, le *N*-[2-(5-{3-[2-(acétylamino)éthyl]-1-benzothien-5-yl}-1*H*-pyrrolo [2,3-*b*]pyridin-3-yl)éthyl]acetamide, ainsi que leurs sels d'addition à un acide ou une base pharmaceutiquement acceptable.

12. Procédé de préparation des composés de formule (I) selon la revendication 1 **caractérisé en ce que** l'on utilise comme produit de départ composé de formule (V) :
A-G₁-Cy-OMe (V)
dans laquelle A, G₁ et Cy sont tels que définis dans la formule (1).
que l'on soumet à une déméthylation en utilisant des agents classiques comme HBr, AlCl₃, AlBr₃, RBr₃ ou des systèmes binaires acide de Lewis / nucléophile comme AlCl₃ / PhCH₂SH ou BBr₃ / Me₂S par exemple, pour obtenir le composé de formule (VI) :
A-G₁-Cy-OH (VI)
dans laquelle A, G₁ et Cy sont définis comme précédemment,
◆ qui est transformé, de façon classique,
- par action du *N,N*-diméthylthiocarbamate de sodium par exemple, en thiol correspondant de formule (VII) :
A-G₁-Cy-SH (VII)
dans laquelle A, G₁ et Cy sont tels que définis précédemment,
- ou en dérivé aminé correspondant de formule (VIII) :
A-G₁-Cy-NHR'ₐ (VIII)
dans laquelle A, G₁ et Cy sont définis comme précédemment et R'ₐ peut prendre toutes les valeurs de Rₐ tel que défini dans la formule (I) et peut également représenter un atome d'hydrogène,
les composés de formule (VI), (VII) et (VIII) représentant le composé de formule (IX) :
A-G₁-Cy-W₄H (IX)
dans laquelle W₄ représente un atome d'oxygène ou de soufre, ou un groupement NH ou NRₐ (où Rₐ est défini comme précédemment),
composé de formule (IX) sur lequel on condense :
• un composé de formule (X) : 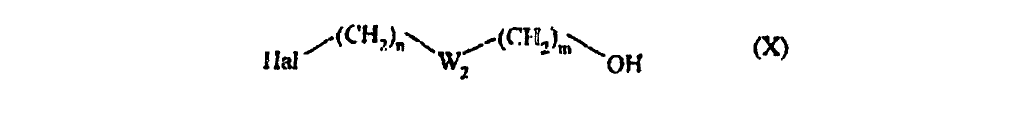 dans laquelle Hal représente un atome de brome, de chlore ou d'iode, et n, W₂ et m sont tels que définis dans la formule (I). (étant entendu que l'on ne peut pas avoir deux hétéroatomes consécutifs et que la chaîne ainsi définie peut comporter une ou plusieurs insaturations).
• ou un composé de formule (XI) : 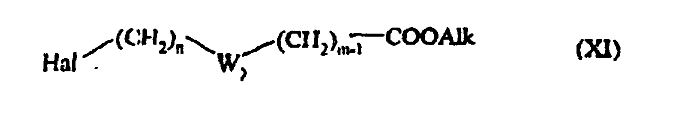 dans laquelle Hal, n, m et W₂ sont définis comme précédemment et Alk représente un radical alkyle (étant entendu que l'on ne peut pas avoir deux hétéroatomes consécutifs et que la chaîne ainsi définie peut comporter une ou plusieurs insaturations), suivi d'une réduction,
pour conduire au composé de formule (XII) :
A-G₁-Cy-W₄-(CH₂)ₙ-W₂-(CH₂)ₘ-OH (XII)
dans laquelle A, G₁, Cy, W₄, n, m et W₂ sont définis comme précédemment (étant entendu que l'on ne peut pas avoir deux hétéroatomes consécutifs dans l'enchaînement -W₄-(CH₂)ₙ-W₂-(CH₂)ₘ-OH et que la chaîne ainsi définie peut comporter une ou plusieurs insaturations),
dont la fonction hydroxyle est transformée classiquement en groupe partant comme par exemple un mésylate, un tosylate, ou un dérivé halogéné, pour conduire au composé de formule (XII') :
A-G₁-Cy-W₄-(CH₂)ₙ-W₂-(CH₂)ₘ-E XIIʹ)
dans laquelle A, G₁, Cy, W₄, n, W₂ et m sont définis comme précédemment et F, représente un groupement mésyle ou tosyle ou un atome d'halogène,
sur lequel on fait agir un composé de formule (XIII) :
B-G₄-Cy'-W'₄H (XIII)
dans laquelle R, G₃ et Cy' sont définis comme dans la formule (I) et W'₄ peut prendre les mêmes valeurs que W₄ défini précédemment,
pour conduire au composé de formule (I/a), cas particulier des composés de formule (I)
A-G₁-Cy-W₄-(CH₂)ₙ-W₂-(CH₂)ₘ-W'₄-Cy'-G₃-B (I/a)
dans laquctle A, G₁, Cy, Cy', W₄, n, W₂, m, W'₄, G₃ et B sont tels que définis précédemment,
• ou transformé en utilisant par exemple le phényl bis (trifluorométhane-sulfonimide) en milieu basique en trifluorométhane sulfonate correspondant de formule (XIV) :
A-G₁-Cy-OSO₂CF₃ (XIV)
dans laquelle A, G₁ ct Cy sont définis comme précédemment,
- sur lequel on fait agir, dans des conditions de catalyse par un dérivé du palladium approprié, un dérivé de l'acide borique (R_{b}B(OH)₂) ou un dérivé de l'étain (R_{b}SnBu₃) (où R_{b} représente un groupement de formule (XV) :
B-G₃-Cy'-W₃-(CH₂)ₘ-W₂-(CH₂)ₙ-CH₂- (XV)
dans laquelle B, G₃, Cy', W₃, m, W₂ et n sont tels que définis précédemment, étant entendu que l'on ne peut pas avoir deux hétéroatomes consécutifs dans l'enchaînement -W₃-(CH₂)ₘ-W₂- et que la chaîne ainsi définie peut comporter une ou plusieurs insaturations),
pour conduire au composé de formule (I/b), cas particulier des composés de formule (I):
A-G₁-Cy-CH₂-(CH₂)ₙ-W₂-(CH₂)ₘ-W₃-Cy'-G₃-B (I/b)
dans laquelle A, G₁, Cy, Cy', n, W₂, m, W₃, G₃ et B sont définis comme précédemment (étant entendu que l'on en peut pas avoir deux hétéroatomes consécutifs dans l'enchaînement -W₂-(CH₂)ₘ-W₃- et que la chaîne ainsi définie peut comporter une ou plusieurs insaturations),
les composés de formule (I/c), cas particulier des composés de formule (I):
A-G₁-Cy-W₁-(CH₂)ₙ-W₂-(CH₂)ₘ-CH₂-Cy'-G₃-B (I/c)
dans laquelle A, G₁, Cy, Cy', W₁, n, W₂, m, G₃ et B sont définis comme précédemment (étant entendu que l'on ne peut pas avoir deux hétéroatomes consécutifs dans l'enchaînement -W₁-(CH₂)ₙ-W₂- et que la chaîne ainsi définie peut comporter une ou plusieurs insaturations),
étant obtenus selon un mode opératoire similaire à partir du composé de formule (XIV') :
B-G₁-Cy'-OSO₂CF₃ (XIVʹ)
dans laquelle B, G₃ et Cy' sont tels que définis précédemment,
- ou que l'on place, dans des conditions de couplage en utilisant des dérivés du Nickel ou du palladium par exemple, en présence d'un composé de formule (XIV') afin de conduire au composé de formule (I/d), cas particulier des composés de formule (I):
A-G₁-Cy-Cy'-G₃-B (I/d)
dans laquelle A, G₁, Cy, Cy', G₃ et B sont tels que définis précédemment.
l'ensemble des composés (I/a) à (I/d) formant le composé de formule (I) qui peut être purifié si on le désire par une technique classique de purification, dont on sépare éventuellement les isomères selon une technique classique de séparation et que l'on transforme, le cas échéant, en leurs sels d'addition à un acide ou à une base pharmaceutiquement acceptable.

13. Compositions pharmaceutiques contenant comme principe actif au moins un composé de formule (I) selon l'une quelconque des revendications 1 à 11 ou un de leurs sels d'addition avec un acide ou une base pharmaceutiquement acceptables en combinaison avec un ou plusieurs excipients pharmaceutiquement acceptables.

14. Compositions pharmaceutiques selon la revendication 13 utiles pour la fabrication d'un médicament pour traiter les troubles liés au système mélatoninergique.

## Claims

1. Compounds of formula (I) :
A-G₁-Cy-G₂-Cy'-G₃-B (I)
wherein:
◆ A represents a grouping of formula 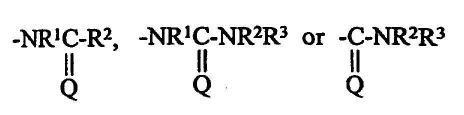 wherein:
- Q represents a sulphur or oxygen atom,
- R¹, R² and R³, which may be identical or different, represent a hydrogen atom or a group Rₐ (wherein Rₐ represents an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)cycloalkyl group, an unsubstituted or substituted cycloalkyl-(C₃-C₈)alkyl group in which the alkyl moiety is linear or branched, a polyhalo-(C₁-C₆)alkyl group in which the alkyl moiety is linear or branched, an aryl group, an aryl-(C₁-C₆)alkyl group in which the alkyl moiety is linear or branched, an aryl-(C₂-C₆)alkenyl group in which the alkenyl moiety is linear or branched, a heteroaryl group, a heteroaryl-(C₁-C₆)alkyl group in which the alkyl moiety is linear or branched or a heteroaryl-(C₂-C₆)alkenyl group in which the alkenyl moiety is linear or branched),
or the groups R² and R³ can also form with the nitrogen atom carrying them a group selected from piperazinyl, piperidinyl and pyrrolidinyl,
◆ B represents a grouping of formula 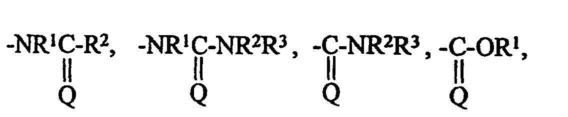 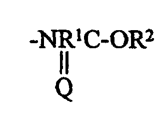 or -NR²R³ wherein Q, R¹, R² and R³ are as defined hereinbefore,
◆ G₁ and G₃, which may be identical or different, represent a linear or branched alkylene chain containing from 1 to 4 carbon atoms that is optionally substituted by one or more identical or different groups selected from hydroxy, carboxy, formyl, Rₐ, ORₐ, COORₐ and CORₐ (wherein Rₐ is as defined hereinbefore),
◆ Cy and Cy', which are different, represent
- a ring structure of formula (II): 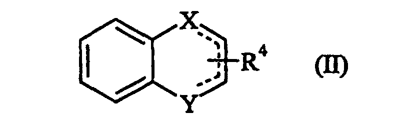 wherein:
* X and Y, which may be identical or different, represent a sulphur, oxygen or carbon atom, or a CH or CH₂ group,
* R⁴ represents a hydrogen or halogen atom, or a CF₃, hydroxy, carboxy, formyl, amino, NHRₐ, NRₐR¹ₐ, NHCORₐ, CONHRₐ, Rₐ, ORₐ, CORₐ or COORₐ group, (wherein Rₐ is as defined hereinbefore and R¹ₐ can have any of the meanings of Rₐ),
* the symbol ---- means that the bonds are single or double, with the proviso that the valency of the atoms is respected,
wherein G₂ substitutes the benzene ring, and G₁ substitutes the ring containing X and Y in the case of Cy,
and G₂ substitutes the benzene ring and G₃ substitutes the ring containing X and Y in the case of Cy',
- or a ring structure of formula (III) : 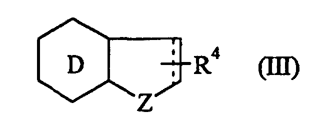
wherein:
* Z represents a sulphur or oxygen atom, or a CH₂, NH, NSO₂Ph or NRₐ group (wherein Rₐ is as defined hereinbefore),
* D represents a benzene or pyridine ring,
* R⁴ is as defined hereinbefore,
* the symbol ---- means that the bond is single or double, with the proviso that the valency of the atoms is respected,
or G₂ substitutes the D ring, and G₁ substitutes the ring containing Z in the case of Cy,
and G₂ substitutes the D ring and G₃ substitutes the ring containing Z in the case of Cy',
the two, different, rings Cy and Cy' of the compounds of formula (I) both being represented by a structure of formula (II) or by a structure of formula (III), or one of the two rings being represented by a structure of formula (II) and the other being represented by a structure of formula (III),
◆ G₂ represents a chain of formula (IV) : 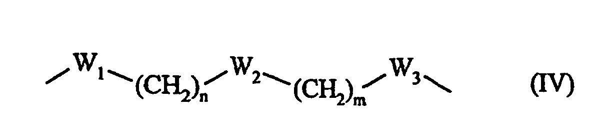 wherein:
- W₁, W₂ and W₃, which may be identical or different, represent a bond, an oxygen or sulphur atom, or a CH₂, CHRₐ, NH or NRₐ group (wherein Rₐ is as defined hereinbefore),
- n represents an integer wherein 0 ≤ n ≤ 6,
- m represents an integer wherein 0 ≤ m < 6,
with the proviso that it is not possible to have two consecutive hetero atoms and that the chain of formula (IV) so defined may have one or more unsaturated bonds,
it being understood that:
- "aryl" is understood to mean the groups naphthyl, phenyl and biphenyl,
- "heteroaryl" is understood to mean any saturated or unsaturated mono- or bicyclic group containing from 5 to 10 members and containing from 1 to 3 hetero atoms selected from nitrogen, sulphur and oxygen,
it being possible for the "aryl" and "heteroaryl" groups to be substituted by one or more identical or different radicals selected from hydroxy, carboxy, linear or branched (C₁-C₆)alkoxy, linear or branched (C₁-C₆)alkyl, polyhalo-(C₁-C₆)-alkyl in which the alkyl moiety is linear or branched, formyl, cyano, nitro, amino, linear or branched. (C₁-C₆)alkylamino, di-(C₁-C₆)alkylamino in which each alkyl moiety is linear or branched, and halogen atoms,
- the term "substituted" applied to the terms "alkyl", "alkenyl" and "alkynyl" means that those groups are substituted by one or more identical or different radicals selected from hydroxy, linear or branched (C₁-C₆)alkoxy, polyhalo-(C₁-C₆)alkyl in which the alkyl moiety is linear or branched, amino, linear or branched (C₁-C₆)alkylamino, di-(C₁-C₆)alkylamino in which each alkyl moiety is linear or branched, and halogen atoms,
- the term "substituted" applied to the terms "cycloalkyl" and "cycloalkylalkyl" means that the cyclic moiety of those groups is substituted by one or more identical or different radicals selected from hydroxy, linear or branched (C₁-C₆)alkoxy, polyhalo-(C₁-C₆)alkyl in which the alkyl moiety is linear or branched, amino, linear or branched (C₁-C₆)alkylamino, di-(C₁-C₆)alkylamino in which each alkyl moiety is linear or branched, and halogen atoms,
their enantiomers and diastereoisomers, and addition salts thereof with a pharmaceutically acceptable acid or base.

2. Compounds of formula (I) according to claim 1 wherein Cy and Cy', which are different, represent a ring structure of formula (II), their enantiomers and diastereoisomers, and addition salts thereof with a pharmaceutically acceptable acid or base.

3. Compounds of formula (I) according to claim 1 wherein Cy and Cy', which are different, represent a ring structure of formula (III), their enantiomers and diastereoisomers, and addition salts thereof with a pharmaceutically acceptable acid or base.

4. Compounds of formula (I) according to claim 1 wherein Cy represents a ring structure of formula (II) and Cy' represents a ring structure of formula (III), their enantiomers and diastereoisomers, and addition salts thereof with a pharmaceutically acceptable acid or base.

5. Compounds of formula (I) according to claim 1 wherein G₂ represents a single bond, their enantiomers and diastereoisomers, and addition salts thereof with a pharmaceutically acceptable acid or base.

6. Compounds of formula (I) according to claim 1 wherein G₂ represents a grouping -W₄-(CH₂)ₚ-W'₄- wherein W₄ and W'₄, which may be identical or different, represent an oxygen or sulphur atom or an NH or NRₐ group, and p represents an integer wherein 1 ≤ p ≤ 12, their enantiomers and diastereoisomers, and addition salts thereof with a pharmaceutically acceptable acid or base.

7. Compounds of formula (I) according to claim 1 wherein G₂ represents a grouping -O-(CH₂)ₚ-O- wherein p represents an integer wherein 1 ≤ p ≤ 12, their enantiomers and diastereoisomers, and addition salts thereof with a pharmaceutically acceptable acid or base.

8. Compounds of formula (I) according to claim 1 wherein A and B, which may be identical or different, represent a grouping NR¹COR² or CONR²R³, their enantiomers and diastereoisomers, and addition salts thereof with a pharmaceutically acceptable acid or base.

9. Compounds of formula (I) according to claim I which are *N*-(2-{7-[2-({3-[2-(acetylamino)ethyl]-1-benzofuran-5-yl}oxy)ethoxy)-1-naphthyl}ethyl)acetamide, *N*-(2-{5-[2-({8-[2-(acetylamino)ethyl]-2-naphthyl}oxy)ethoxy]-1-benzofuran-3-yl}ethyl)-2-furamide, *N*-(2-{5-[2-({8-[2-(acetylamino)ethyl]-2-naphthyl}oxy)ethoxy]-*1H*-pyrrolo-[*2,3-b*]pyridin-3-yl}ethyl)cyclopropanecarboxamide, *N*-(2-{7-[3-({3-[2-(acetylamino)-ethyl]-1-benzothiophen-5-yl}oxy)propoxy]-1-naphthyl}ethyl)acetamide, *N*-[2-(5-{[6-({8-[2-(acetylamino)ethyl]-2-naphthyl}oxy)hexyl]oxy}-*1H*-pyrrolo[2,3-*b*]pyridin-3-yl)ethyl]-acetamide, *N*-(2-{5-[4-({3-[2-(acetylamino)ethyl]-1-benzothien-5-yl}oxy)butoxy]-1*H*-indol-3-yl}ethyl)acetamide, *N*-(2-{5-[4-({3-[2-(acetylamino)ethyl]-1-benzothien-5-yl}-oxy)butoxy]-1*H*-pyrrolo[2,3-*b*]pyridin-3-yl}ethyl)acetamide, *N*-(2-{5-[4-({3-[2-(acetyl-amino)ethyl]-1-benzofuran-5-yl}oxy)butoxy]-1*H*-pyrrolo[2,3*-b*]pyridin-3-yl}ethyl)-acetamide, *N*-(2-{5-[4-({3-[2-(acetylamino)ethyl]-1*H*-indol-5-yl}oxy)butoxy]-1*H*-pyrrolo[2,3-*b*]pyridin-3-yl}ethyl)acetamide, and addition salts thereof with a pharmaceutically acceptable acid or base.

10. Compounds of formula (I) according to claim 1 which are *N*-(2-{7-[4-({3-[2-(acetylamino)ethyl]-1-benzofuran-5-yl}oxy)butoxy]-1-naphthyl}ethyl)acetamide, *N*-{2-[5-[4-({8-[2-(acetylamino)ethyl]-2-naphthyl}oxy)butoxy]-1-(phenylsulphonyl)-1*H*-indol-3-yl]ethyl}acetamide, *N*-(2-{7-[4-({8-[2-(acetylamino)ethyl]-2-naphthyl}oxy)butoxy]-1,2, 3,4-tetrahydro-1-naphthalenyl}ethyl)acetamide, *N*-(2-{5-[4-({3-[2-(acetylamino)ethyl]-1-benzofuran-5-yl}oxy)butoxy]-1*H*-indol-3-yl}ethyl)acetamide, *N*-(2-{7-[4-({3-[2-(acetylamino)ethyl]-1-benzothien-5-yl}oxy)butoxy]-1-naphthyl}ethyl)acetamide, and addition salts thereof with a pharmaceutically acceptable acid or base.

11. Compounds of formula (I) according to claim 1 which are *N*-[2-(7-{3-[2-(acetylamino)ethyl]-1-benzofuran-5-yl}-1-naphthyl)ethyl]acetamide, *N*-[3-(5-{8-[2-(acetylamino)ethyl]-2-naphthyl}-*1H*-pyrrolo[*2,3-b*]pyridin-3-yl)propyl]heptanamide, *N*-[2-(7-{3-[2-(acetylamino)ethyl]-1-benzothien-5-yl}-1-naphthyl)ethyl]acetamide, *N*-[2-(5-{8-[2-(acetylamino)ethyl]-2-naphthyl}-1*H*-pyrrolo[2,3-*b*]pyridin-3-yl)ethyl]acetamide, *N*-[2-(5-{3-[2-(acetylamino)ethyl]-1-benzofuran-5-yl}-1-benzothien-3-yl)ethyl]acetamide, *N*-[2-(5-{3-[2-(acetylamino)ethyl]-1-benzofuran-5-yl}-1*H*-indol-3-yl)ethyl]acetamide, *N*-[2-(5-{3-[2-(acetylamino)ethyl]-1-benzofuran-5-yl}-1*H*-pyrrolo[2,3-*b*]pyridin-3-yl)ethyl]-acetamide, *N*-[2-(5-{3-[2-(acetylamino)ethyl]-1*H*-indol-5-yl}-1*H*-pyrrolo[2,3-*b*]pyridin-3-yl)ethyl]acetamide, *N*-[2-(5-{3-[2-(acetylamino)ethyl]-1-benzothien-5-yl}-1*H*-pyrrolo-[2,3-*b*]pyridin-3-yl)ethyl]acetamide, and addition salts thereof with a pharmaceutically acceptable acid or base.

12. Process for the preparation of compounds of formula (I) according to claim 1, **characterised in that** there is used as starting material a compound of formula (V):
A-G₁-Cy-OMe (V)
wherein A, G₁ and Cy are as defined for formula (I),
which is subjected to demethylation using conventional agents, such as, HBr, AlCl₃, AlBr₃, BBr₃ or Lewis acid / nucleophile binary systems, such as, for example, AlCl₃ / PhCH₂SH or BBr₃ / Me₂S, to obtain a compound of formula (VI):
A-G₁-Cy-OH (VI)
wherein A, G₁ and Cy are as defined hereinbefore,
◆ which is converted, in conventional manner,
- by the action of, for example, sodium *N,N*-dimethylthiocarbamate, to the corresponding thiol of formula (VII):
A-G₁-Cy-SH (VIII)
wherein A, G₁ and Cy are as defined hereinbefore,
- or to the corresponding amine compound of formula (VIII)
A-G₁-Cy-NHR'ₐ (VIII)
wherein A, G₁ and Cy are as defined hereinbefore and R'ₐ can have any of the meanings of Rₐ as defined for formula (I) and can also represent a hydrogen atom,
which compounds of formulae (VI), (VII) and (VIII) represent the compound of formula (IX):
A-G₁-Cy-W₄H (IX)
wherein W₄ represents an oxygen or sulphur atom, or an NH or NRₐ group (wherein Rₐ is as defined hereinbefore),
which compound of formula (IX) is condensed with :
• a compound of formula (X): 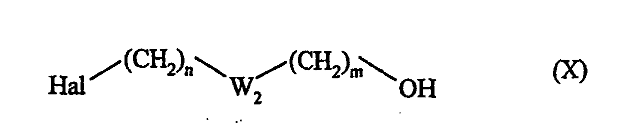 wherein Hal represents a bromine, chlorine or iodine atom, and n, W₂ and m are as defined for formula (I), (with the proviso that it is not possible to have two consecutive hetero atoms and that the chain so defined may have one or more unsaturated bonds),
• or a compound of formula (XI) : 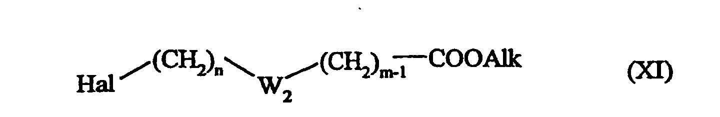 wherein Hal, n, m and W₂ are as defined hereinbefore and Alk represents an alkyl radical (with the proviso that it is not possible to have two consecutive hetero atoms and that the chain so defined may have one or more unsaturations), followed by reduction,
to yield a compound of formula (XII) :
A-G₁-Cy-W₄-(CH₂)ₙ-W₂-(CH₂)ₘ-OH (XII)
wherein A, G₁, Cy, W₄, n, m and W₂ are as defined hereinbefore (with the proviso that it is not possible to have two consecutive hetero atoms in the -W₄-(CH₂)ₙ-W₂-(CH₂)ₘ-OH chain and that the chain so defined may have one or more unsaturated bonds),
the hydroxyl function of which is converted in conventional manner to a leaving group, such as, for example, a mesylate, a tosylate, or a halogen compound, to yield a compound of formula (XII'):
A-G₁-Cy-W₄-(CH₂)ₙ-W₂-(CH₂)ₘ-E (XII')
wherein A, G₁, Cy, W₄, n, W₂ and m are as defined hereinbefore and E represents a mesyl or tosyl group or a halogen atom,
which is subjected to the action of a compound of formula (XIII) :
B-G₃-Cy'-W'₄H (XIII)
wherein B, G₃ and Cy' are as defined for formula (I) and W'₄ can have the same meanings as W₄ defined hereinbefore,
to yield a compound of formula (I/a), a particular case of the compounds of formula (I):
A-G₁-Cy-W₄-(CH₂)ₙ-W₂-(CH₂)ₘ-W'₄-Cy'-G₃-B (I/a)
wherein A, G₁, Cy, Cy', W₄, n, W₂, m, W'₄, G₃ and B are as defined hereinbefore,
◆ or converted using, for example, phenyl bis(trifluoromethanesulphonimide) in a basic medium to the corresponding trifluoromethanesulphonate of formula (XIV) :
A-G₁-Cy-OSO₂CF₃ (XIV)
wherein A, G₁ and Cy are as defined hereinbefore,
- which is subjected, under conditions of catalysis by a suitable palladium compound, to the action of a boric acid compound (R_{b}B(OH)₂) or of a tin compound (R_{b}SnBu₃) (wherein R_{b} represents a grouping of formula (XV):
B-G₃-Cy'-W₃-(CH₂)ₘ-W₂-(CH₂)ₙ-CH₂- (XV)
wherein B, G₃, Cy', W₃, m, W₂ and n are as defined hereinbefore, with the proviso that it is not possible to have two consecutive hetero atoms in the -W₃-(CH₂)ₘ-W₂- chain and that the chain so defined may have one or more unsaturated bonds),
to yield a compound of formula (I/b), a particular case of the compounds of formula (I) :
A-G₁-Cy-CH₂-(CH₂)ₙ-W₂-(CH₂)ₘ-W₃-Cy'-G₃-B (I/b)
wherein A, G₁, Cy, Cy', n, W₂, m, W₃, G₃ and B are as defined hereinbefore (with the proviso that it is not possible to have two consecutive hetero atoms in the -W₂-(CH₂)ₘ-W₃- chain and that the chain so defined may have one or more unsaturated bonds),
which compounds of formula (I/c), a particular case of the compounds of formula (I) :
A-G₁-Cy-W₁-(CH₂)ₙ-W₂-(CH₂)ₘ-CH₂-Cy'-G₃-B (I/c)
wherein A, G₁, Cy, Cy', W₁, n, W₂, m, G₃ and B are as defined hereinbefore (with the proviso that it is not possible to have two consecutive hetero atoms in the -W₁-(CH₂)ₙ-W₂- chain and that the chain so defined may have one or more unsaturated bonds),
are obtained according to a similar procedure starting from a compound of formula (XIV'):
B-G₃-Cy'-OSO₂CF₃ (XIV')
wherein B, G₃ and Cy' are as defined hereinbefore,
- or is treated, under coupling conditions using, for example, nickel or palladium compounds, with a compound of formula (XIV') to yield a compound of formula (I/d), a particular case of the compounds of formula (I):
A-G₁-Cy-Cy'-G₃-B (I/d)
wherein A, G₁, Cy, Cy', G₃ and B are as defined hereinbefore,
the totality of the compounds (I/a) to (I/d) constituting the compounds of formula (I) which may be purified, if desired, by a conventional purification technique, are separated, where appropriate, into their isomers according to a conventional separation technique, and converted, if necessary, into addition salts thereof with a pharmaceutically acceptable acid or base.

13. Pharmaceutical compositions comprising as active ingredient at least one compound of formula (I) according to any one of claims 1 to 11 or an addition salt thereof with a pharmaceutically acceptable acid or base in combination with one or more pharmaceutically acceptable excipients.

14. Pharmaceutical compositions according to claim 13 for use in the production of a medicament for the treatment of disorders associated with the melatoninergic system.

## Patentansprüche

1. Verbindungen der Formel (I):
A-G₁-Cy-G₂-Cy'-G₃-B (I)
in der:
◆ A eine Gruppe der Formel darstellt 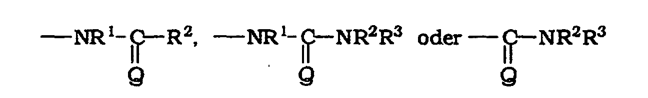 in denen:
- Q ein Schwefel- oder Sauerstoffatom bedeutet,
- R¹, R² und R³, die gleichartig oder verschieden sind, ein Wasserstoffatom oder eine Gruppe Rₐ (worin Rₐ eine geradkettige oder verzweigte, nichtsubstituiert oder substituierte (C₁-C₆)-Alkylgruppe, geradkettige oder verzweigte, substituierte oder nichtsubstituierte (C₂-C₆)-Alkenylgruppe, geradkettige oder verzweigte, nichtsubstituierte oder substituierte (C₂-C₆)-Alkinylgruppe, substituierte oder nichtsubstituierte (C₃-C₈)-Cycloalkylgruppe, geradkettige oder verzweigte, nichtsubstituierte oder substituierte (C₃-C₈)-Cycloalkylalkylgruppe, geradkettige oder verzweigte (C₁-C₆)-Polyhalogenalkylgruppe, Arylgruppe, geradkettige oder verzweigte Aryl-(C₁-C₆)-alkylgruppe, geradkettige oder verzweigte Aryl-(C₂-C₆)-alkenylgruppe, Heteroarylgruppe, geradkettige oder verzweigte Heteroaryl-(C₁-C₆)-alkylgruppe oder geradkettige oder verzweigte Heteroaryl-(C₂-C₆)-alkenylgruppe darstellt), bedeuten, oder die Gruppen R² und R³ zusammen mit dem sie tragenden Stickstoffatom eine Gruppe bilden können ausgewählt aus Piperazinyl, Piperidinyl oder Pyrrolidinyl,
◆ B eine Gruppe der Formel bedeutet 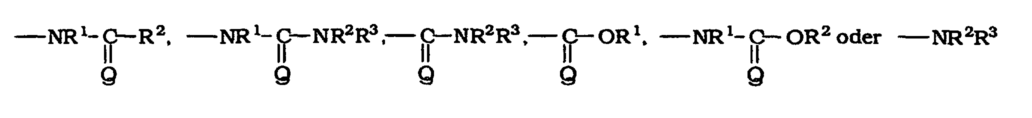 in der Q, R¹, R² und R³ die oben angegebenen Bedeutungen besitzen,
◆ G₁ und G₃, die gleichartig oder verschieden sind, eine geradkettige oder verzweigte Alkylenkette mit 1 bis 4 Kohlenstoffatomen darstellen, die gegebenenfalls durch eine oder mehrere gleichartige oder verschiedene Gruppen ausgewählt aus Hydroxy, Carboxy, Formyl, Rₐ, ORₐ, COORₐ oder CORₐ (worin Rₐ die oben angegebenen Bedeutungen besitzt) substituiert ist,
◆ Cy und Cy', die unterschiedlich sind
- eine cyclische Struktur der Formel (II): 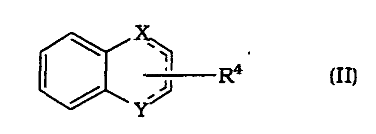 in der:
* X und Y, die gleichartig oder verschieden sind, ein Schwefelatom, ein Sauerstoffatom oder ein Kohlenstoffatom oder eine Gruppe CH oder CH₂ darstellen,
* R⁴ ein Wasserstoffatom, ein Halogenatom oder eine CF₃-Gruppe, Hydroxygruppe. Carboxygruppe, Formylgruppe, Aminogruppe oder Gruppe der Formel NHRₐ, NRₐR¹ₐ, NHCORₐ, CONHRₐ, Rₐ, ORₐ, CORₐ oder CO-ORₐ (worin Rₐ die oben angegebenen Bedeutungen besitzt und R¹ₐ sämtliche Bedeutungen von Rₐ annehmen kann), bedeutet,
* das Symbol ---- bedeutet, daß die Bindungen einfach oder doppelt sind,
mit der Maßgabe, daß die Wertigkeit der Atome respektiert ist,
wobei im Fall von Cy G₂ den Benzolring substituiert und G₁ den Ring substituiert, der X und Y enthält,
und im Fall von Cy' G₂ den Benzolring substituiert und G₃ den Ring substituiert. der X und Y enthält,
- oder eine cyclische Struktur der Formel (III) bedeuten: 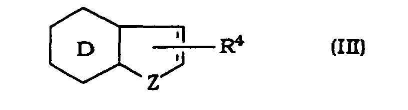 in der:
* Z ein Schwefelatom, ein Sauerstoffatom oder eine Gruppe CH₂, NH, NSO₂Ph oder NRₐ (worin Rₐ die oben angegebenen Bedeutungen besitzt) darstellt,
* D einen Benzol- oder Pyridin-Ring bedeutet.
* R⁴ die oben angegebenen Bedeutungen besitzt und
* das Symbol ---- bedeutet, daß die Bindung einfach oder doppelt ist, mit der Maßgabe, daß die Wertigkeit der Atome respektiert ist,
im Fall von Cy G₂ den Ring D substituiert und G₁ den Ring substituiert, der Z enthält
und im Fall von Cy' G₂ den Ring D substituiert und G₃ den Ring substituiert, der Z enthält,
wobei die beiden Ringe Cy und Cy' der Verbindungen der Formel (I), die unterschiedlich sind, jeweils beide durch eine Struktur der Formel (II) repräsentiert werden, beide durch eine Struktur der Formel (III) repräsentiert werden oder eine durch die Struktur der Formel (II) und die andere durch eine Struktur de Formel (III).
◆ G₂ eine Kette der Formel (IV) bedeutet: 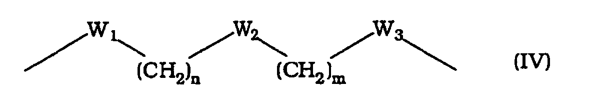 in der:
- W₁, W₂ und W₃, die gleichartig oder verschieden sind, eine Bindung, ein Sauerstoffatom, ein Schwefelatom oder eine Gruppe CH₂, CHRₐ, NH oder NRₐ (worin Rₐ die oben angegebenen Bedeutungen besitzt) darstellen,
- n eine ganze Zahl entsprechend der Beziehung 0 ≤ n ≤ 6 darstellt,
- m eine ganze Zahl entsprechend der Beziehung 0 ≤ m ≤ 6 bedeutet,
mit der Maßgabe, daß keine zwei aufeinanderfolgenden Heteroatome vorliegen und daß die in dieser Weise definierte Kette der Formel (IV) eine oder mehrere Unsättigungen aufweisen kann,
wobei es sich versteht, daß man:
- unter «Aryl» Naphthyl-, Phenyl- und Biphenyl-gruppen versteht,
- unter «Heteroaryl» jede mono- oder bicyclische, gesättigte oder ungesättigte Gruppe versteht, die 5 bis 10 Kettenglieder aufweist und 1 bis 3 Heteroatome ausgewählt aus Stickstoff, Schwefel oder Sauerstoff enthält,
wobei die « Arylgruppen » und die « Heteroarylgruppen » durch einen oder mehrere gleichartige oder verschiedene Reste ausgewählt aus Hydroxy, Carboxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Formyl. Cyano, Nitro, Amino, geradkettigem oder verzweigtem (C₁-C₆)-Alkylamino, geradkettigem oder verzweigtem (C₁-C₆)-Dialkylamino oder Halogenatomen substituiert sein können,
- der Begriff «substituiert», bezogen auf die Begriffe «Alkyl», «Alkenyl» und «Alkinyl», bedeutet, daß diese Gruppe durch einen oder mehrere gleichartige oder verschiedene Reste ausgewählt aus Hydroxy, geradkettigem oder verzweigtem (C₁-C₆)-Alkoxy, geradkettigem oder verzweigtem (C₁-C₆)-Polyhalogenalkyl, Amino, geradkettigem oder verzweigtem (C₁-C₆)-Alkylamino, geradkettigem oder verzweigtem (C₁-C₆)-Dialkylamino oder Halogenatomen substituiert sind.
- der Begriff «substituiert», bezogen auf die Begriffe «Cycloalkyl» und «Cycloalkylalkyl» bedeutet, daß der cyclische Teil dieser Gruppen durch einen oder mehrere gleichartige oder verschiedene Reste ausgewählt aus Hydroxy, geradkettigem oder verzweigtem (C₁-C₆)-Alkoxy, geradkettigem oder verzweigtem (C₁-C₆)-Polyhalogenalkyl, Amino, geradkettigem oder verzweigtem (C₁-C₆)-Alkylamino, geradkettigem oder verzweigtem (C₁-C₆)-Dialkylamino oder Halogenatomen substituiert ist,
deren Enantiomere und Diastereoisomere sowie deren Additionssalze mit einer pharmazeutisch annehmbaren Säure oder Base.

2. Verbindungen der Formel (I) nach Anspruch 1, worin Cy und Cy', die verschiedenartig sind, eine cyclische Struktur der Formel (II) darstellen, deren Enantiomere und Diastereoisomere sowie deren Additionssalze mit einer pharmazeutisch annehmbaren Säure oder Base.

3. Verbindungen der Formel (I) nach Anspruch 1, worin Cy und Cy', die verschiedenartig sind, eine cyclische Struktur der Formel (III) darstellen, deren Enantiomere und Diastereoisomere sowie deren Additionssalze mit einer pharmazeutisch annehmbaren Säure oder Base.

4. Verbindungen der Formel (I) nach Anspruch 1, worin Cy eine cyclische Struktur der Formel (II) und Cy' eine cyclische Struktur der Formel (III) darstellen, deren Enantiomere und Diastereoisomere sowie deren Additionssalze mit einer pharmazeutisch annehmbaren Säure oder Base.

5. Verbindungen der Formel (I) nach Anspruch 1, worin G₂ eine Einfachbindung bedeutet, deren Enantiomere und Diastereoisomere sowie deren Additionssalze mit einer pharmazeutisch annehmbaren Säure oder Base.

6. Verbindungen der Formel (I) nach Anspruch 1, worin G₂ eine Gruppe -W₄-(CH₂)ₚ-W'₄- darstellt, worin W₄ und W'₄, die gleichartig oder verschieden sind, ein Wasserstoffatom, ein Schwefelatom oder eine Gruppe NH oder NRₐ und p eine ganze Zahl entsprechend der Beziehung 1 ≤ p ≤ 12 bedeuten, deren Enantiomere und Diastereoisomere sowie deren Additionssalze mit einer pharmazeutisch annehmbaren Säure oder Base.

7. Verbindungen der Formel (I) nach Anspruch 1, worin G₂ eine Gruppe -O-(CH₂)ₚ-O- darstellt, worin p eine ganze Zahl entsprechend der Beziehung 1 ≤ p ≤ 12 darstellt, deren Enantiomere und Diastereoisomere sowie deren Additionssalze mit einer pharmazeutisch annehmbaren Säure oder Base.

8. Verbindungen der Formel (I) nach Anspruch 1, worin A und B, die gleichartig oder verschieden sind, eine Gruppe NR¹COR² oder CONR²R³ darstellen, deren Enantiomere und Diastereoisomere sowie deren Additionssalze mit einer pharmazeutisch annehmbaren Säure oder Base.

9. Verbindungen der Formel (I) nach Anspruch 1, nämlich N-(2-{7-(2-({3-(2-Acetylamino)-ethyl]-1-benzofuran-5-yl}-oxy)-ethoxy]-1-naphthyl}-ethyl)-acetamid, *N*-(2-{5-[2-({8-[2-(Acetylamino)-ethyl]-2-naphthyl}-oxy)-ethoxy]-1-benzofuran-3-yl}-ethyl)-2-furamid, *N*-(2-{5-[2-({8-[2-(Acetylamino)-ethyl]-2-naphthyl}-oxy)-ethoxyl-1*H*-pyrrolo[2,3-*b*]pyridin-3-yl}-ethyl-cyclopropancarboxamid, *N*-(2-{7-[3-({3-[-2-(Acetylamino)-ethyl]-1-benzothiophen-5-yl}-oxy)-propoxy]-1-naphthyl}-ethyl)-acetamid, *N*-[2-(5-{[6-({8-[2-(Acetylamino)-ethyl]-2-naphthyl}-oxy}hexyl]-oxy}-*1H*-pyrrolo[2,3-*b*]pyridin-3-yl)-ethyl]-acetamid, *N*-(2-{5-[4-({3-[2-Acetylamino]-ethyl]-1-benzothien-5-yl}-oxy)-butoxyl-1*H*-indol-3-yl}-ethyl)-acetamid, *N*-(2-{5-[4-({3-[2-(Acetylamino]-ethyl]-1-benzothien-5-yl}-oxy)-butoxy]-1H-pyrrolo[2,3-*b*]pyridin-3-yl}ethyl-acetamid, *N*-(2-{5-[4-({3-[2-(Acetylamino)-ethyl]-1-benzofuran-5-yl}-oxy)-butoxy]-1*H*-pyrrolo[2,3-*b*]pyridin-3-yl}-ethyl)-acetamid, *N*-(2-{5-[4-({3-[2-(Acetylamino)-ethyl]-1*H*-indol-5-yl}-oxy)-butoxy]-1*H*-pyrrolo[2,3-*b*]pyridin-3-yl}-ethyl)-acetamid sowie deren Additionssalze mit einer pharmazeutisch annehmbaren Säure oder Base.

10. Verbindungen der Formel (I) nach Anspruch 1, nämlich N-(2-{7-[4-({3-[2-(Acetylamino)-ethyl]-1-benzofuran-5-yl}-oxy)-butoxyl-1-naphthyl}-ethyl)-acetamid, *N*-(2-[5-[4-({8-(2-(Acetylamino)-ethyl]-2-naphthyl}-oxy)-butoxy]-1-(phenylsulfonyl)-1*H*-indol-3-yll-ethyl}-acetamid, *N*-(2-{7-(4-({8-[2-(Acetylamino)-ethyl]-2-naphthyl}-oxy)-butoxy]-1,2,3,4-tetrahydro-1-naphthalinyl}-ethyl)-acetamid, N-(2-{5-[4-({3-[2-(Acetylamino)-ethyl]-1-benzofuran-5-yl}-oxy)-butoxy]-1*H*-indol-3-yl}-ethyl)-acetamid, *N*-(2-{7-[4-({3-[2-(Acetylamino)-ethyl]-1-benzothien-5-yl}-oxy)-butoxy]-1-naphthyl}-ethyl)-acetamid sowie deren Additionssalze mit einer pharmazeutisch annehmbare Säure oder Base.

11. Verbindungen der Formel (I) nach Anspruch 1, nämlich *N*-[2-(7-{3-[2-[Acetylamino)-ethyl]-1-benzofuran-5-yl}-1-naphthyl)-ethyl]-acetamid, *N*-[3-(5-{8-[2-(Acetylamino)-ethyl]-2-naphthyl}-1*H-*pyrrolo[2,3-*b*]pyridin-3-yl)-propyl]-heptanamid, *N*-[2-(7-{3-[2-(Acetylamino)-ethyl]-1-benzothien-5-yl}-1-naphthyl)-ethyl]-acetamid, *N*-[2-(5-{8-[2-(Acetylamino)-ethyl]-2-naphthyl}-1*H*-pyrrolo[2,3-*b*]pyridin-3-yl)-ethyl]-acetamid. *N*-[2-(5-{3-[2-(Acetylamino)-ethyl]-1-benzofuran-5-yl}-1-benzothien-3-yl)-ethyl]-acetamid, *N*-[2-(5-{3-[2-(Acetylamino)-ethyl]-1-benzofuran-5-yl}-1*H*-indol-3-yl)-ethyl]-acetamid, *N*-[2-(5-{3-[2-(Acetylamino)-ethyl]-1-benzofuran-5-yl}-1*H*-pyrrolo[2,3-*b*]pyridin-3-yl)-ethyl]-acetamid, *N*-[2-(5-{3-[2-(Acetylamino)-ethyl]-1*H*-indol-5-yl}-1*H*-pyrrolo[2, 3-*b* ]pyridin-3-yl)-ehyl]-acetamid, *N*-[2-(5-{3-[2-(Acetylamino)-ethyl]-1-benzothien-5-yl}-1*H*-pyrrolo(2,3-*b*]pyridin-3-yl)-ethyl]-acetamid sowie deren Additionssalze mit einer pharmazeutisch annehmbaren Säure oder Base.

12. Verfahren zur Herstellung der Verbindungen der Formel (I) nach Anspruch 1, **dadurch gekennzeichnet, daß** man als Ausgangsprodukt eine Verbindung der Formel (V) verwendet:
A - G₁ - Cy - OMe (V)
in der A, G₁ und Cy die bezüglich der Formel (I) angegebenen Bedeutungen besitzen.
welche man einer Demethylierung unterwirft unter Verwendung klassischer Mittel. wie HBr, AlCl₃, AlBr₃, BBr₃ oder binäre Lewis-Säure/Nucleophil-Systeme, wie beispielsweise AlCl₃/PhCH₂SH oder BBR₃/Me₂S, zur Bildung der Verbindung der Formel (VI):
A - G₁ - Cy - OH (VI)
in der A, G1 und Cy die oben angegebenen Bedeutungen besitzen,
◆ welche man in klassischer Weise
- durch Einwirkung von beispielsweise Natrium-*N,N*-dimethylthiocarbamat in das entsprechende Thiol der Formel (VII):
A - G₁ - Cy - SH (VII)
in der A, G₁ und Cy die oben angegebenen Bedeutungen besitzen,
- oder in das entsprechende Amin-Derivat der Formel (VIII):
A - G₁ - Cy - NHR'ₐ (VIII)
in der A, G₁ und Cy die oben angegebenen Bedeutungen besitzen und R'ₐ sämtliche Bedeutungen von Rₐ annehmen kann, wie sie oben definiert worden sind und auch ein Wasserstoffatom darstellen kann, umwandelt,
wobei die Verbindungen der Formeln (VI), (VII) und (VIII) die Verbindung der Formel (IX) darstellen:
A-G₁-Cy-W₄H (IX)
in der W₄ ein Sauestoffatom oder ein Schwefelatom oder eine Gruppe NH oder NRₐ (worin Rₐ die oben angegebenen Bedeutungen besitzt) darstellt, welche Verbindung der Formel (IX) man:
• mit einer Verbindung der Formel (X) kondensiert: 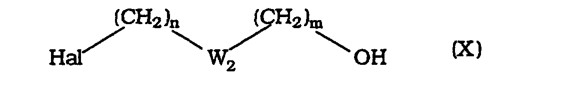 in der Hal ein Bromatom, ein Chloratom oder ein Iodatom darstellt und n, W2 und m die bezüglich der Formel (I) angegebenen Bedeutungen besitzen (wobei es sich versteht, daß nicht zwei aufeinanderfolgende Heteroatome vorliegen und daßdie in dieser Weise definierte Kette eine oder mehrere Unsättigungen aufweisen kann),
• oder mit einer Verbindung der Formel (XI) kondensiert: 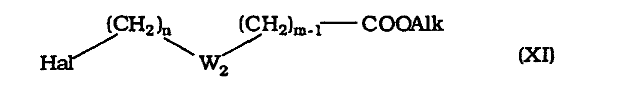 in der Hal, n, m un W₂ die oben angegebenen Bedeutungen besitzen und Alk eine Alkylgruppe darstellt (wobei es sich versteht, daß nicht zwei aufeinanderfolgende Heteroatome vorliegen und daß die in dieser Weise definierte Kette eine oder mehrere Unsättigungen aufweisen kann), gefolgt von einer Reduktion
zur Bildung der Verbindung der Formel (XII):
A-G₁-Cy-W₄-(CH₂)ₙ-W₂-(CH₂)ₘ-OH (XII)
in der A, G₁, Cy, W₄, n, m und W₂ die oben angegebenen Bedeutungen besitzen (wobei es sich versteht, daß nicht zwei aufeinanderfolgende Heteroatome in der Kette W₄-(CH₂)ₙ-W₂-(CH₂)ₘ-OH vorliegen und daß die in dieser Weise definierte Kette eine oder mehrere Unsättigungen aufweisen kann), deren Hydroxylfunktion in klassischer Weise in eine austretende Gruppe, beispielsweise eine Mesylatgruppe oder eine Tosylatgruppe oder ein Halogenderivat umgewandelt wird zur Bildung der Verbindung der Formel (XII'):
A-G₁-Cy-W₄-(CH₂)ₙ-W₂-(CH₂)ₘ-E (XII')
in der A, G₁, Cy, W₄, n, W₂ und m die oben angegebenen Bedeutungen besitzen und E eine Mesyl- oder Tosylgruppe oder ein Halogenatom darstellt, welche man mit einer Verbindung der Formel (XIII) umsetzt:
B-G₃-Cy'-W'₄H (XIII)
in der B, G₃ und Cy' die oben bezüglich der Formel (I) angegebenen Bedeutungen besitzen und W'₄ sämtliche Bedeutungen für W₄, wie sie oben definiert worden sind, annehmen kann, zur Bildung der Verbindung der Formel (I/a), einem Sonderfall der Verbindungen der Formel (I):
A-G₁-Cy-W₄-(CH₂)ₙ-W₂-(CH₂)ₘ-W'₄-Cy'-G₃-B (I/a)
in der A, G₁, Cy, Cy', W₄, n, W₂, m, W'₄, G₃ und B die oben angegebenen Bedeutungen besitzen,
◆ welche man unter Verwendung von beispielsweise Phenyl-bis(trifluormethansulfonimid) in basischem Medium in das entsprechende Trifluormethansulfonat der Formel (XIV) umwandelt:
A-G₁-Cy-OSO₂CF₃ (XIV)
in der A, G₁ und Cy die oben angegebenen Bedeutungen besitzen,
- welche man unter katalytischen Bedingungen eines geeigneten Palladiumderivats mit einem Borsäurederivat (R_{b}B(OH)₂) oder einem Zinnderivat (R_{b}SnBu₃) (worin R_{b} eine Gruppe der Formel (XV) darstellt:
B-G₃-Cy'-W₃-(CH₂)ₘ-W₂-(CH₂)ₙ-CH₂₋ (XV)
in der B, G₃, Cy', W₃, m, W₂ und n die oben angegebenen Bedeutungen besitzen, wobei es sich versteht, daß keine zwei aufeinanderfolgenden Heteroatome in der Kette -W₃-(CH₂)ₘ-W₂- vorliegen können und daß die in dieser Weise definierte Kette eine oder mehrere Unsättigungen aufweisen kann), umsetzt zur Bildung der Verbindung der Formel (I/b), einem Sonderfall der Verbindungen der Formel (I):
A-G₁-Cy-CH₂-(CH₂)ₙ-W₂-(CH₂)ₘ-W₃-Cy'-G₃-B (I/b)
in der A, G₁, Cy, Cy', W₄, n, W₂, m, W₃, G₃ und B die oben angegebenen Bedeutungen besitzen (wobei es sich versteht, daß keine zwei aufeinanderfolgenden Heteroatome ind er Kette -W₂-(CH₂)ₘ-W₃- vorliegen können unddaß die in dieser Weise definierte Kette eine oder mehrere Unsättigungen aufweisen kann),
wobei man die Verbindungen der Formel (I/c). einem Sonderfall der Verbindungen der Formel (I):
A-G₁-Cy-W₁-(CH₂)ₙ-W₂-(CH₂)ₘ-CH₂-Cy'-G₃-B (I/c)
in der A, G₁, Cy, Cy', W₁, n, W₂, m, G₃ und B die oben angegebenen
Bedeutungen besitzen (wobei es sich versteht, daß keine zwei aufeinanderfolgenden Heteroatome in der Kette -W₁-(CH₂)ₙ-W₂- vorliegen können und daß die in dieser Weise definierte Kette eine oder mehrere Unsättigungen aufweisen kann),
man in einer ähnlichen Weise erhält, ausgehend von der Verbindung der Formel (XIV'):
B-G₃-Cy'-OSO₂CF₃ (XIV')
in der B, G₃ und Cy' die oben angegebenen Bedeutungen besitzen,
- oder welche man unter Kupplungsbedingungen unter Verwendung von beispielsweise Nickel- oder Palladium-Derivaten mit einer. Verbindung der Formel (XIV') umsetzt zur Bildung der Verbindung der Formel (I/d), einem Sonderfall der Verbindungen der Formel (I):
A-G₁-Cy-Cy'-G₃-B (I/d)
in der A, G₁, Cy, Cy', G₃ und B die oben angegebenen Bedeutungen besitzen,
wobei die Gesamtheit der Verbindungen (I/a) bis (I/d) die Verbindung der Formel (I) bilden, welche gewünschtenfalls mit Hilfe einer klassischen Reinigungsmethode gereinigt werden kann, welche gegebenenfalls mit Hilfe einer klassischen Trennmethode in ihre Isomeren getrennt werden kann und welche gegebenenfalls in ihre Additionssalze mit einer pharmazeutisch annehmbaren Säure oder Base umgewandelt werden kann.

13. Pharmazeutische Zubereitungen enthaltend als Wirkstoff mindestens eine Verbindung der Formel (I) nach einemd er Ansprüche 1 bis 11 oder eines ihrer Additionssalze mit einer pharmazeutisch annehmbaren Säure oder Base in Kombination mit enem oder mehreren pharmazeutisch annehmbaren Trägermaterialien.

14. Pharmazeutische Zubereitungen nach Anspruch 13 nützlich für die Herstellung eines Arzneimittels zur Behandlung von Störungen, die mit dem melatoninergischen System verknüpft sind.
